# EUROPEAN PATENT SPECIFICATION

(11) **EP 2 726 633 B1**
(45) Date of publication and mention of the grant of the patent: **24.01.2018**
(21) Application number: 12804520.0
(22) Date of filing: 02.07.2012
(51) Int. Cl.: C12Q 1/68, G01N 33/53

(54) **PROCESS AND APPARATUS FOR QUANTIFYING NUCLEIC ACID IN A SAMPLE**
VERFAHREN UND VORRICHTUNG ZUR QUANTIFIZIERUNG VON NUKLEINSÄUREN IN EINER PROBE
PROCÉDÉ ET APPAREIL PERMETTANT DE QUANTIFIER UN ACIDE NUCLÉIQUE DANS UN ÉCHANTILLON

(30) Priority: 30.06.2011 US 201161503599 P
(43) Date of publication of application: 07.05.2014
(73) Proprietor: BIOQUANTA SA, 75003 Paris (FR); BIOQUANTA, CORP., Aurora, CO 80014 (US); ASSISTANCE PUBLIQUE - HOPITAUX DE PARIS, 75001 Paris (FR)
(72) Inventor: CONTI, Marc, 91120 Palaiseau (FR); LORIC, Sylvain, 91800 Brunoy (FR); MANIVET, Philippe, 75012 Paris (FR); DELACOTTE, Nicolas, 94240 L' Hay-les Roses (FR); KEUMEUGNI KWEMO, Carlosse, 92390 Villeneuve La Garenne (FR); SALIOU BAH, Mamadou, 92250 La Garenne Colombes (FR)
(74) Representative: Ponzellini, Gianmarco
(86) International application number: PCT/US2012/045271
(87) International publication number: WO 2013/003849

(56) References cited:
- WO-A1-93/06241
- WO-A2-03/078966
- WO-A2-2004/057016
- WO-A2-2010/129787
- US-A- 4 921 805
- US-A1- 2004 157 217
- US-A1- 2010 216 145
- PAWLOTSKY ET AL: "What technique should be used for routine detection and quantification of HBV DNA in clinical samples?", JOURNAL OF VIROLOGICAL METHODS, ELSEVIER BV, NL, vol. 65, no. 2, 1 May 1997 (1997-05-01), pages 245-253, XP005538376, ISSN: 0166-0934, DOI: 10.1016/S0166-0934(97)02196-4

## Description

### FIELD OF THE INVENTION

The invention relates to processes that ensure an easy measurement of even a small quantity of nucleic acids, without the need for sample extraction, purification, or amplification. The invention is adapted to various kinds of readout methods. The processes are suitable for manual handling, semi-automation or automation. The present processes are suitable for a fully automated operation, and can be carried out in one or several vessels, containers or wells, without further handling of the samples or the reaction components or the entire reaction mixture, with only an eventual transfer of reacting material by automated means from one vessel to another.

The invention further relates to a method for quantifying cell-free nucleic acids, i.e., nucleic acids present outside cells. It finds use, inter alia, in ascertaining the existence of an abnormality characterized by the incidence of cell free nucleic acid in the bloodstream of a patient, and in monitoring a patient previously presenting with such an abnormality and after such patient has been treated to detect possible recurrence of the abnormality.

### BACKGROUND OF THE INVENTION

After cellular disruption, such as apoptosis, nuclear DNA spills into the extracellular environment and is carried by the blood. This DNA is referred to as cell-free DNA (or free DNA) and its existence has been known for many years (Mandel & Métais, 1948; Tan *et al.*, 1966; Koffer *et al.*, 1973). Healthy individuals typically have about 500-1000 genome equivalents of free DNA per milliliter of blood. A value in this range corresponds to a normal rate of cellular disruption. A higher level may be an indication of a disorder that clinicians have to explore and treat. Accordingly, accurate, fast, relatively low-cost and convenient measurement of cell-free DNA is desirable.

To date, high concentrations of cell-free DNA have been correlated with various pathologies (*e.g*., lung cancer, ovarian cancer, breast cancer, melanoma, adrenal hyperplasia, cervical cancer, preeclampsia, fetal Down's syndrome, nasopharyngeal carcinoma, leukemia, gastrointestinal malignancy, lupus erythematosus, rheumatoid arthritis); consequently, cell-free DNA is beginning to be used as a marker for these diseases.

In addition, the quantification of cell-free DNA in plasma can be used to predict the likelihood of serious cardiovascular complications, which permits appropriate therapy to be given at an early stage in an attempt to lower the incidence of such complications.

The detection and quantification of cell-free DNA has also added a new dimension to medical diagnosis. In oncology, various tumor-associated molecular alterations have been detected in the plasma/serum of cancer patients. In pregnant subjects, the discovery of fetal DNA in maternal circulating blood has provided a new source of fetal genetic material for the noninvasive analysis of numerous fetal conditions, and for the detection of particular pregnancy-associated disorders. The measurement of cell-free DNA has also found potential applications in the posttreatment monitoring of transplant patients and in the assessment and prognosis of trauma patients.

Radioimmunoassay techniques have historically been employed to detect high concentrations of cell free DNA in patients with cancer, as compared to healthy subjects, or subjects with benign tumors (Leon *et al.*, 1977; Shapiro *et al.*, 1983). Numerous studies have reported that high concentrations of cell free DNA can serve as a diagnostic marker or of progression of breast or lung cancer, or could be useful in the follow-up of patients treated by chemotherapy.

In addition, sub-physiological concentration of cell free DNA may signify an altered pathology, which may require medical treatment adjustment.

Despite the fact that non-physiological concentrations of cell-free DNA were known to be correlated with various disease states, including cancer, cell-free DNA has not been used as a biological marker until the advent of quantitative PCR (qPCR). A main reason for this is that radioactive assays, although able to detect cell free DNA, were not optimal due to accompanying safety concerns and lack of both sensitivity and convenience of implementation.

Sensitive, non-invasive techniques such as qPCR allow the clinician to avoid, or limit, more invasive tests like tissue biopsies for the detection of cancer. Assaying cell free DNA content by qPCR can also serve as a screen, at an early stage of lung, breast or prostate cancer. Moreover, the measurement of cell-free DNA content by qPCR can be used as a complementary technique to that of classical marker detection (*e.g*., by cell sorting methodologies) in the follow-up of post-operative patients, patients with pathology (Chang *et al.*, 2003), or patients diagnosed or treated for cancer (Lam *et al.*, 2003).

Although qPCR is extremely sensitive in terms of detection limit, it has several practical and technical drawbacks. Careful consideration has to be paid to reaction set-up, including reagent concentrations, contamination from external sources, and purity of sample. False positives can arise if the reaction components and conditions are not diligently controlled. Crude samples usually need to be purified before their use in a qPCR assay to remove non-nucleic acid components of the sample which inhibit the amplification reaction or quench the reporter molecule's fluorescence. Since the amplification procedure is exponential, small differences in reaction component concentration (*e.g.*, polymerase, fluorescent probe, salt, etc.) that may exist from reaction-to-reaction, can lead to large differences in the amount of template detected, even while keeping the template concentration constant.

The majority of nucleic acid quantification methods (*e.g.,* radioimmunological methods, qPCR, and other fluorometric detection methods, such as ELISA) require the use of purified nucleic acid samples as templates for the quantification assays. In the case of ELISA, antibodies can bind to non-nucleic acid components of the sample. In the case of qPCR, *Taq* polymerase activity can be inhibited by components of the crude sample.

Using the methods described above, it typically takes at least 3 hours for the entire process of DNA isolation/purification and quantification to be completed. The time period necessary for nucleic acid quantification by current methodologies limits these tools for use in point-of-care diagnostics and on-site forensic applications.

Work on cell-free nucleic acids started in 1948 but the last ten years haven given rise to 80% of the publications on the subject, reflecting a growing interest and need in this diagnostic marker. There is a particular need for a rapid automated process to measure cell-free DNA that could be performed on the available machines used in clinical diagnostic services. And this need is not fulfilled with the qPCR methods developed.

A viable alternative for the detection of DNA in crude samples is the use of a luminescent readout. Superior sensitivity, wide dynamic range, low instrument cost and low background are all characteristics of luminescent methods. For example, luminometry is up to 100,000 times more sensitive than absorption spectroscopy and is at least 1,000 times more sensitive than fluorometry. The unmatched sensitivity of luminescent reactions allows quantification of nucleic acids without amplification steps.

In addition, safety, ease of handling and significantly lower disposal costs are advantages of luminescent assays over analogous radiography methods.

Luminescent assays require simpler instrumentation as compared to fluorescence assays because there is no need for a light excitation source. Luminescence can be quantified since light output is directly proportional to the number of luminescent substrates in the reaction (when volume is held constant from reaction-to-reaction). Undesirable autofluorescence, background emission and scatter are eliminated with the use of luminescence because no excitation light is necessary to generate a signal. In luminescent reactions, temperature and extraneous light must be controlled for since both affect reaction rates.

Although luminescent assays afford the advantages listed above, they have not been coupled to DNA purification and subsequent quantification in one integrated assay. A main reason for this is the inability to accurately quantify luminescence in an environment containing components other than the target that is to be quantified. The present invention sets out to solve this problem with an integrated purification/quantification assay.

If the luminescence is an easy way to quantify cell-free nucleic acids, it is possible to envisage other readout methods, once nucleic acid is captured from the sample by an interactor or preferably by two or more interactors. So we propose to integrate in the same general process, capture, extraction and measurement of cell-free nucleic acids. Capture and extraction will be done by the interactor, and measure of the signal emitted by a label excited or otherwise revealed at the end of the process. The signal has to be sensitive enough to ensure measurement of small amounts of cell free nucleic acids. It may be, without limitations, optic (UV, Visible, IR absorption), fluorescent, luminescent or magnetic. US 4921805 discloses a method of quantifying HBV DNA comprising a separation of nucleic acids from a sample by unspecific capture to a solid support carrying a nucleic acid intercalator followed by a release of bound DNA, the blotting of the released nucleic acids onto a membrane a hybridisation with radio labelled HBV specific RNA probes followed by a quantification of the hybridisation signal.

### SUMMARY OF THE INVENTION

The present invention serves as an alternative to amplification-based nucleic acid detection methods which require prior nucleic acid purification/isolation. The present invention also integrates the processes of purification and detection of nucleic acids. In some embodiments, the present disclosure provides detection of cell free nucleic acids in a sample without amplification and/or isolation of nucleic acids in the sample.

The present invention is suitable for manual handling or semi-automated or automated processes.

In case of automation, processes can be carried out during a unique fully automated process, in one or several vessels, containers or wells, with automatic transfer of reacting material by the machine or another machine.

In one aspect, the present invention integrates capture, labeling, detection and quantification of nucleic acids in a sample, and can be carried out in one automated process and in a single vessel.

The present invention can be carried out with various existing or novel automatic analyzers.

Our data indicated that the interaction between cell-free nucleic acids and the interactor was dependent on both the sequence and length of the nucleic acids. Thus, the use of one kind of interactor is not enough to be sure to measure all cell-free nucleic acids contained in a sample. A set of two (or more) interactors, such as three or four, etc., is preferably utilized in the methods described herein. An integrated procedure that both isolates and quantifies nucleic acids from a crude sample, with the use of various readout methods, is disclosed herein. The methods provided, as compared to nucleic acid purification/detection schemes previously developed (*e.g.,* DNA isolation by chromatography followed by qPCR), are compatible with automation, reduce reagent cost, overall reaction set-up time, as well as time for detection, because nucleic acids are not amplified, or transferred to a second reaction zone after isolation.

In one aspect, the present invention concerns a method for quantifying a target nucleic acid in a sample by sequestration of the target nucleic acid by one or more interactors to form a sequestered conjugate that will be further labeled to form a labeled conjugate. The signal produced by the labeled conjugate is measured and correlated to the amount of the target nucleic acid. In another aspect, the present invention concerns a method for quantifying a target nucleic acid in a sample by a reaction of the nucleic acids in the sample with a nucleic acid interactor or set of interactors to form a conjugate. The conjugate is then sequestered from the rest of the sample with a molecule bound to a support, to form a sequestered conjugate. The sequestered conjugate is labeled to form a labeled conjugate. The signal produced by the labeled conjugate is measured and correlated to the amount of the target nucleic acid. In some embodiments, the target nucleic acid is cell free nucleic acids. In embodiments, the interactor binds to nucleic acids in the sample in a nonsequence specific manner.

In yet another aspect, the present invention concerns a method for quantifying a target nucleic acid in a sample introducing a known quantity of a nucleic acid labeled with a readable molecule into the sample containing the target nucleic acid. The target nucleic acid and the labeled nucleic acid are sequestered by a sequestered nucleic acid interactor (or set of interactors) to form a sequestered target conjugate and a sequestered labeled conjugate wherein the target competes with the labeled nucleic acid for binding to the interactor. The signal of the labeled conjugate is then measured. The amount of target nucleic acid is then derived by comparing the signal of the labeled conjugate in the presence of target nucleic acid with that emitted by sequestered labeled conjugate in the absence of target nucleic acid.

In some embodiments, a method for quantifying a target nucleic acid in a sample comprises:(a)contacting the target nucleic acid in the sample with a sequestered nucleic acid interactor to form a sequestered conjugate; (b) adding at least one more nucleic acid interactor; (c) labeling the sequestered conjugate with a signal generating molecule to form a labeled conjugate; and (d)measuring the signal of the labeled conjugate; wherein the signal of the labeled conjugate is indicative of the amount of the target nucleic acid in the sample. In embodiments, the method further comprises comparing the amount of the target nucleic acids to a control sample to determine if the amount of target nucleic acids of the sample is indicative of disease. In embodiments, a method further comprises communicating the result to a health care provider so that the health care provider can conduct additional diagnostic tests. In some embodiments, the target nucleic acid is cell free nucleic acids. In embodiments, the interactor binds to nucleic acids in the sample in a nonsequence specific manner.

In other embodiments, a method for quantifying a target nucleic acid in a sample comprises:(a) contacting the nucleic acid in the sample with at least one nucleic acid interactor to form a conjugate; (b) sequestering the conjugate by reacting the conjugate with a capture molecule bound to a support;(c)labeling the sequestered conjugate with a signal generating molecule to form a labeled conjugate;(d) measuring signal of the labeled conjugate; wherein the signal of the labeled conjugate is indicative of the amount of the target nucleic acid in the sample. In some embodiments, the method further comprises comparing the number or amount of the target nucleic acids to a control sample to determine if the number or amount of target nucleic acids of the sample is indicative of disease or disorder. In some embodiments, a method further comprises communicating the result to a health care provider so that the health care provider can conduct additional diagnostic tests. In some embodiments, the target nucleic acid is cell free nucleic acids. In embodiments, the interactor binds to nucleic acids in the sample in a nonsequence specific manner.

In yet other embodiments, a method for quantifying a target nucleic acid in a sample comprises:(a) providing a known quantity of nucleic acid labeled with a signal generating molecule into the sample containing the target nucleic acid;(b) reacting the target nucleic acid and the labeled nucleic acid with a sequestered nucleic acid interactor to form a sequestered target conjugate and a sequestered labeled conjugate, wherein the target nucleic acid competes with the labeled nucleic acid for binding to said sequestered interactor; (c) measuring the signal of the sequestered labeled conjugate; and(d) determining the amount of said target nucleic acid by comparing the signal in step (c) with that detected by sequestered labeled conjugate in the absence of target nucleic acid. In embodiments, the method further comprises comparing the amount of the target nucleic acids to a control sample to determine if the amount of target nucleic acids of the sample is indicative of disease or disorder. In embodiments, a method further comprises communicating the result to a health care provider so that the health care provider can conduct additional diagnostic tests. In some embodiments, the target nucleic acid is cell free nucleic acids. In embodiments, the interactor binds to nucleic acids in the sample in a nonsequence specific manner.

In a specific embodiment, a method for quantifying a target nucleic acid in a sample comprises: contacting the target nucleic acid in the sample with a sequestered nucleic acid interactor to form a sequestered conjugate; contacting the sequestered conjugate with a second nucleic acid interactor labeled with a signal generating element to form a labeled conjugate; and measuring signal of the labeled conjugate; wherein the signal of the labeled conjugate is indicative of the amount of the target nucleic acid in the sample. In some embodiments, the method further comprises comparing the amount of the target nucleic acids to a control sample to determine if the amount of target nucleic acids of the sample is indicative of disease or disorder. In some embodiments, a method further comprises communicating the result to a health care provider so that the health care provider can conduct additional diagnostic tests. In some embodiments, the target nucleic acid is cell free nucleic acids. In embodiments, the interactor binds to nucleic acids in the sample in a nonsequence specific manner.

Another aspect of the disclosure provides a kit for quantifying a target nucleic acid in a sample comprising (i) a first nucleic acid interactor; (ii) a second nucleic acid interactor that is labeled with a readable label and which interactor is different from said first nucleic acid interactor; (iii) a label, optionally bound to the first interactor; and (iv) instructions for use.

Since the interaction between cell-free nucleic acids and a single interactor was dependent on both the sequence and length of the nucleic acids, the use of one kind of interactor may not be enough to ensure the measurement of all cell-free nucleic acids contained in the medium. So the invention preferably uses two or more interactors to improve the accuracy of the process.

In one aspect, the nucleic acid to be quantified in the sample is cell-free DNA.

The foregoing methods are useful, inter alia, in ascertaining the existence of an abnormality characterized by the incidence of cell free nucleic acid in the bloodstream of a patient, and in monitoring a patient previously presenting with such an abnormality (after such patient has been subjected to therapy to abate or eliminate the abnormality) to detect possible recurrence of the abnormality. When these methods are employed, upon the finding of an abnormality, further tests can be employed, such as DNA assays known in the art, to determine the nature of the abnormality. Thus, embodiments of the invention contemplate performance of the methods disclosed herein in tandem with sequence specific DNA detection methods.

### DRAWINGS

Fig. 1A is a general diagram of a sandwich assay embodiment of the invention, showing target nucleic acid free in solution (top) and sequestered (bottom).
Fig. 1B is a general diagram of a competitive assay embodiment of the invention showing target nucleic acid free in solution (top) and sequestered (bottom).
Fig 2A-C is a general diagram of an embodiment in which a capture system, linked to the support via the interactor with the label binding to the captured nucleic acid, is used.
Fig 3A-C is a general diagram of an embodiment in which a capture system, linked to the support via the label, is used.
Fig 4A-B contain a graph illustrating the variation of fluorescence signal intensity with DNA segment length (in base pairs). Several sequences of variable lengths were tested, at the same concentration (20 ng/ml), with either A) Picogreen or B) Popo3 as intercalating agent. Fluorescence using a single interactor to bind the nucleic acid appears to be length dependent.
Fig 5A-E are plots of fluorescence vs concentration of DNA for DNA of different base pair lengths. Each sequence (X1 to X5 ; A-E) is tested, with Picogreen, at several concentrations. In each case, fluorescence is well correlated with concentration.
Fig. 6 is a plot of fluorescence vs. sequence identity. Several different sequences of comparable lengths were tested, at the same concentration (20 ng/ml), with a single intercalating agent: either Picogreen ◆ or Popo3 ■. Fluorescence using a single interactor appears to be sequence dependent.
Fig.. 7 A-B are respectively a plot of fluorescence vs DNA segment length (panel A) or sequence type (panel B) Several sequences of A) variable lengths or B) variable sequence identity, were tested, at the same concentration (20 ng/ml), with a mix of four intercalating agents : picogreen, popo 3, yoyo 1, orange acridin, at equimolar ratios. Fluorescence appears to be no longer length-or sequencedependent.

### DETAILED DESCRIPTION OF CERTAIN EMBODIMENTS

### Definitions

A "support," for use with the present invention, is any solid phase which can serve as a substrate for immobilizing the first nucleic acid capture system. In some embodiments, the support is the wall or a well, *e.g.*, a well in a 96-well plate, or a wall of a well fabricated into a glass or quartz slide. In certain embodiments, the support is a tip. In other embodiments the support is glass or quartz, *e.g.*, a microscope slide, coverslip, or a portion thereof. In some embodiments, the support is a particle, bead or molecule that can be concentrated in solution (*e.g.,* paramagnetic particles, streptavidin...). In other embodiments, the support is an inner wall of a reaction vessel. In particular embodiments the support is paper, cellulose or nitrocellulose, or any derivatives. In short any type of solid support can be used.

A "vessel," for use with the present invention, can be any tube (*e.g.,* 0.2 mL, 0.5 mL, 1 mL, 1.5 mL, and 2 mL in volume), container, or well, which has an opening and is enclosed on all other sides. Multiple wells can be joined together to form a plate, such as a 96-well plate or any other plate adapted to be used in an automated equipment. In terms of the present invention, a support consisting of glass or quartz (as described above) is also considered a vessel. In some embodiments the vessel is a tip.

A "linker," or "linking moiety," for use with the present invention, is any molecule, set of molecules (*e.g.*, binding partners, such as biotin/streptavidin, antibody/antigen), or chemical bond, which has served to connect either (1) the support and the first capture system, (2) each of the first and second capture system with the label (sandwich assay embodiments), (3) a competing nucleic acid and a label (competitive method embodiments), or (4) a label and a support (one labeled capture system method). In some embodiments, the linker is a peptide, a single stranded nucleic acid, a carbon chain, or simply a covalent bond. In other embodiments, the linker is an antibody or nucleic acid binding molecule. In some embodiments, the linker is composed of a plurality of molecules.

A "linking entity", as used herein, is one of the units that is attached to the linker. For example, a linker can have a support and capture system as linking entities.

A "target" or "target nucleic acid", as used herein, is a nucleic acid molecule to be isolated and quantified by the present invention, and is present in a sample. The target can be either single or double stranded nucleic acid. In some embodiments, the target is cell-free nucleic acid such as DNA, and in some more specific embodiments double-stranded cell-free DNA.

The term "nucleic acid", as used herein, encompasses both single-stranded and double-stranded nucleic acid molecules. The "nucleic acid" can be a DNA or RNA molecule, as well as a DNA/RNA chimera, a nucleic acid molecule with unnatural bases and/or sugar moieties, or a nucleic acid within another kind of molecule chimera. The nucleic acid, when double stranded, has a minimum length of 10 base pairs. In the case of single stranded molecules, 10 nucleotides is the minimum length.

"Cell free DNA", as used herein, is a DNA molecule originally present within the nucleus of an intact cell, which is free in an extracellular environment, after spontaneous or induced cell lysis or disruption. In some embodiments, "cell free DNA" is extracted, purified or amplified nucleic acid.

A "genome equivalent", as used herein, is the amount of DNA originally present in one intact cell.

The "sample" used in the present invention can be any medium which contains the target nucleic acid in a non-cellular (*i.e.,* an extracellular or other cell-free) environment. For example, blood, plasma or serum or any other biological sample (*e.g.,* CSF, urine) can be used as samples for the present invention. In some embodiments, the sample is a cell lysate. In some embodiments, samples may be obtained from a purification, concentration, amplification or dilution process. In some embodiments, samples may be man-made, such as a media effluent from an industrial process. In other embodiments, the sample is a mix of natural and man-made fluids. In various embodiments, samples may be beverages, perfumes, food, or any type of other fluid that could contain free nucleic acids.

A "nucleic acid interactor" or "nucleic acid interacting molecule", as used herein, refers to a molecule that binds to nucleic acids with specificity to ensure subsequent steps of the process. The interactor is therefore capable of capturing and/or isolating (*i.e.*, sequestering) a target nucleic acid from a sample. Nucleic acid interactors include, without limitation, nucleic acid intercalating agents (also referred to as intercalators), as well as minor and major groove binders, small molecules which bind nucleic acid in a non-sequence-specific manner, non-sequence-specific nucleic acids, fusion molecules resulting from a combination of several molecules or from a set of molecules and single stranded nucleic acids (*e.g.,* peptide-nucleic acid chimera) and antibodies, or any type of other combination. These molecules are used as the capture system to initially sequester the target nucleic acid. In some embodiments of the invention, a nucleic acid interactor can act as both an intercalating agent and a minor or major groove binder.

In various embodiments, the "nucleic acid interactor" results from a combination of nucleic acid interactors, to compensate the sequence- or length-dependence of the interaction between the specific interactor and the target nucleic acid. The set of interactors ensures measurement of all nucleic acids contained in the medium.

A "conjugate" or "conjugated nucleic acid", as used herein, is a target nucleic acid bound to a nucleic acid interactor. In various embodiments, a conjugated nucleic acid is a double stranded nucleic acid or triple stranded nucleic acid bound to a nucleic acid interactor. In embodiments where the target nucleic acid is single stranded, a conjugate can refer to the double stranded sequestered molecule, where one strand is the target molecule and the other strand is part of the capture system or permits the capture system to operate by forming a double stranded conjugate.

A "sequestered conjugate", as used herein, refers to a target nucleic acid bound to a nucleic acid interactor, wherein the conjugate is also bound to a support.

A "capture system" or "capture molecule," as used herein, is a compound or set of compounds used to bind and/or sequester the target nucleic acid. The present invention makes use of at least a first capture system, to sequester the target nucleic acid (herein, "capture molecule 1," "capture system 1" or "first capture system"). In the sandwich method, a second capture system is employed (herein, "capture molecule 2", "capture system 2" or "second capture system") to further bind the sequestered nucleic acid. Capture system 2 is used for the sandwich assay embodiment and is labeled with a readable molecule directly or through a linker. In some embodiments, the second capture system is linked to a molecule that may react with a binding partner linked to a label directly or through a linker.

In some embodiments, a capture system (first or second capture system) can include as a component a single stranded or a set of single stranded non sequence-specific nucleic acid intended to form a double stranded nucleic acid or triple stranded nucleic acid with the target, a nucleic acid interactor, a nucleic acid binding molecule such as a small molecule or a transcription factor, or a chromosomal protein or a combination of them. In certain embodiments, the capture system may be labeled, directly or indirectly, or not labeled. In various embodiments, the capture system may become readable once linked to the conjugate or the sequestered conjugate. In certain embodiments, DNA or RNA or more generally nucleic acid primers or nucleic acids having sequences complementary to the target nucleic acid (such that the recognition is sequence-specific) are excluded from the capture system.

In various embodiments, the capture system binds the target nucleic acid by non-covalent interactions. Some non-covalent interactions that can be employed are hydrogen bonding, cation- π, π - π interactions, ionic pairing, hydrophobic interaction, dipole-dipole, dipole-induced-dipole, charge-dipole and van Der Waals interactions. In certain embodiments, the capture system comprises antibodies which bind epitopes common to most or all double stranded nucleic acids. For example, antibodies generated in patients with various autoimmune diseases can be harnessed. See Tzioufas AG, Manoussakis MN, Drosos AA et al. "Enzyme immunoassays for the detection of IgG and IgM anti-dsDNA antibodies: clinical significance and specificity." Clin Exp Rheumatol. 1987; 5:247-253.

A "label", as used in the present invention, is any moiety which can be used in the readout reaction and which binds the target nucleic acid or competing nucleic acid, through a linker (competitive assay) or through an additional capture system and linker (sandwich assay). In certain embodiments the label may be linked, directly or indirectly to the first capture system.

In certain embodiments, in which luminescence is used as the readout method, the label will be the moiety from which light is emitted. In some embodiments the label can be acridinium, ruthenium, Europium, XL665 or dioxetane.

In various embodiments, the label is a particle detectable in a magnetic field (e.g. magnetic, ferromagnetic paramagnetic or superparamagnetic beads (Dynabeads)).

The signal emitted by the label can be optic (UV, Visible, IR absorption), fluorescent, luminescent or magnetic, it may be detected without limitations by NMR, circular dichroism, or Raman spectroscopy.

In certain embodiments, the label is an enzyme that modifies its substrate so as to be detected by any means cited *supra,* including luminescence.

A "labeled conjugate", as used herein, is a conjugate, sequestered or otherwise, further bound in some manner to a label. A "sequestered labeled conjugate," refers to a labeled conjugate further bound to a support.

"Essentially exclusively labeling a sequestered conjugate", as used herein, means binding a label to a sequestered conjugate, directly or indirectly. Non-specific binding of the label to components other than sequestered conjugates may occur, but the signal generated from these interactions can be subtracted from the total signal and, therefore, false positive signals will not be counted. The non-specific labeling can be quantified by a calibration step before the method is carried out. In some embodiments, the ratio of the label bound to a sequestered conjugate as compared to other components in the reaction is 1000:1, 500:1, 100:1 or 10:1. In some embodiments, the ratio is 5:1 or 2:1.

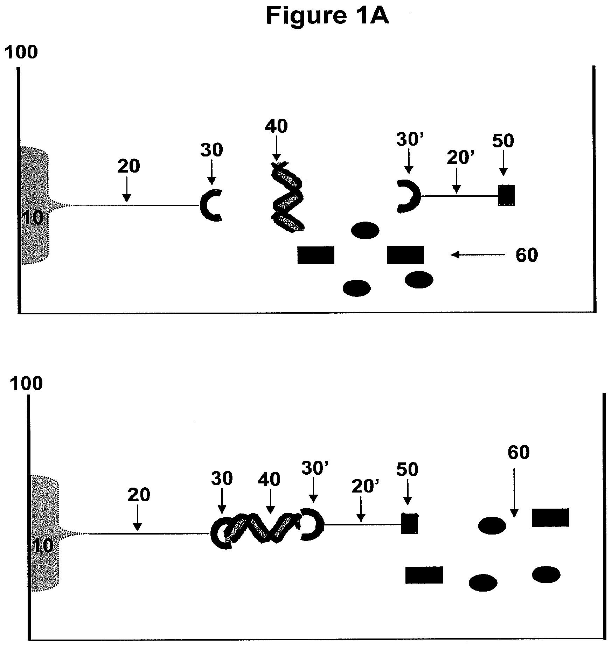

In some embodiments, when a conjugate is "essentially exclusively labeled," the labeling starts in solution, *i.e.,* the support is not labeled with the readable molecule prior to the addition of target nucleic acid (the support is not "prelabeled"). The earliest the label can associate *(i.e.,* indirectly bind, *see* Fig. 1A) with the support is at the time the target nucleic acid binds the first capture system. The first capture system is the reaction component that will either be bound to the support originally (*see* Fig. 1A), or will bind the support after introduction into the reaction. The label cannot indirectly bind the support without the first capture system and the nucleic acid molecule (*see* Fig. 1A).

In some embodiments, the label may be linked, directly or indirectly, to the first capture system and link itself to the support, directly or indirectly. In this case, the capture system binds the nucleic acid, in a non-sequence-specific manner, and there is no need for a second step to bind the label, already linked, via the capture system, to the conjugate. In certain embodiments, the entity formed by the capture system and the label may become readable once the capture system binds to the target, or once the label links itself to the conjugate (see Fig 1C)

In some embodiments, the label may be linked, directly or indirectly, to the support. It may be linked, directly or indirectly, to the capture system. In this case the capture system binds the nucleic acid, in a non-sequence-specific manner, and there is no need for a second step to bind the label, already linked, via the capture system, to the conjugate. In certain embodiments the entity formed by the capture system and the label may become readable once capture system binds to the target, or once the label links itself to the conjugate (see Fig 1D)

The term "particles detectable in a magnetic field" or "magnetic particles", as used herein, is intended to encompass all particles (*e.g*., beads or irregular shape particles) that can capture (*i.e.,* interact with, or associate with) cells or nucleic acids or both (under different buffer conditions). Included are ferromagnetic, paramagnetic, and superparamagnetic particles, which are commercially available through companies such as Promega and Invitrogen.

A "competitive assay" or "competitive method", as used herein, is an assay in which a known concentration of labeled nucleic acid molecules competes with the target nucleic acid (present in the sample) for binding to the capture system. The amount of label detected is inversely proportional to the amount of target nucleic acid originally present in the sample. See U.S. Patent No. 5,198,368 for a detailed description of competitive fluorescence/luminescence assays.

A "sandwich assay" or "sandwich method", as used herein, is an assay in which target nucleic acid binds to the capture system 1, therefore sequestering the nucleic acid to the support. The target nucleic acid is quantified by binding a second capture system to the sequestered nucleic acid, which is labeled, and by detecting the quantity of label.

A "one labeled capture system method", as used herein, is an assay in which the first capture system contains a label. Once the target nucleic acid is bound the sequestered capture system, the label becomes active. The amount of the label detected is proportional to the amount of target nucleic acid present in the sample.

A "luminescent reaction", as used herein, is any reaction in which light is produced. There may or may not be an electron excitation step, by light directly or by fluorescence resonance energy transfer (FRET, see *e.g*., Patent No WO 2010/129787), to generate the light (*e.g.,* a fluorescence based assay). When no electron excitation takes place, light is generated by either a chemiluminescent or a bioluminescent reaction (*e.g*., luciferase-mediated oxidation, acridium ester with hydrogen peroxide, alkaline phosphatase with dioxetane, dioxetane with *β* - galactosidase, horseradish peroxidase with luminol and gold mixed with luminol, phosphatase and ombelliferone, electrochemical excitation of ruthenium and tripropylamine, or UV excitation of Europium).

### Specific Embodiments

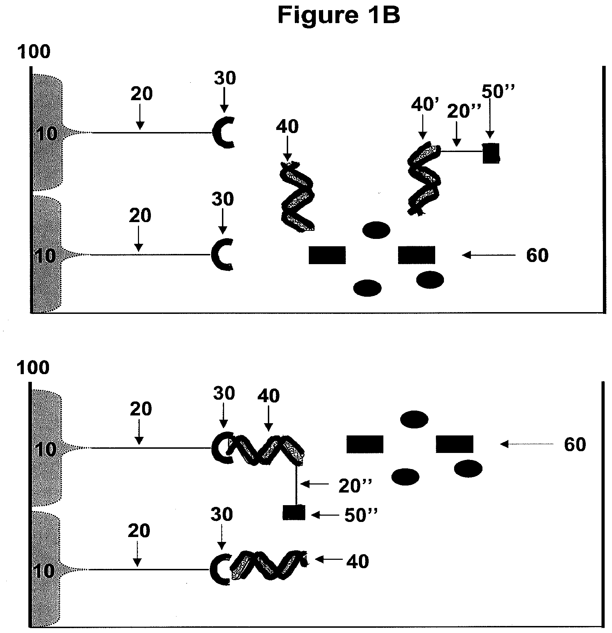

By way of overview and introduction, **Figs. 1A** and **1B** provide schematics of two classes of embodiments of the invention. **Fig. 1A** represents the sandwich assay embodiment. A reaction vessel **100** contains a support **10,** linked to a first capture system e.g., (a first intercalator or other nucleic acid interactor **30** through a linker **20).** The support can be, for example, the inner vessel wall or a magnetic particle. The first capture system **30** discriminately sequesters the target nucleic acid **40** from a crude sample **60,** containing impurities. In some embodiments, a second capture system **30'** (*i.e.,* a second nucleic acid interactor) is added to the reaction and binds the sequestered target nucleic acid. The second capture system **30'** is linked to a readable label **50** through a linker **20'.**

Figure 1B shows a schematic of a general competitive assay embodiment of the invention. A reaction vessel **100** contains a support **10,** linked to a first capture system **30** through a linker **20.** The support can be, for example, the inner vessel wall or a magnetic particle. The capture system **30** discriminately sequesters the target nucleic acid **40** from a crude sample **60,** containing impurities. The target nucleic acid **40** competes with a known concentration of labeled nucleic acid **40'** for binding to the capture system. The known quantity of labeled nucleic acid **40'** is linked to a readable label **50"** through a linker **20".**

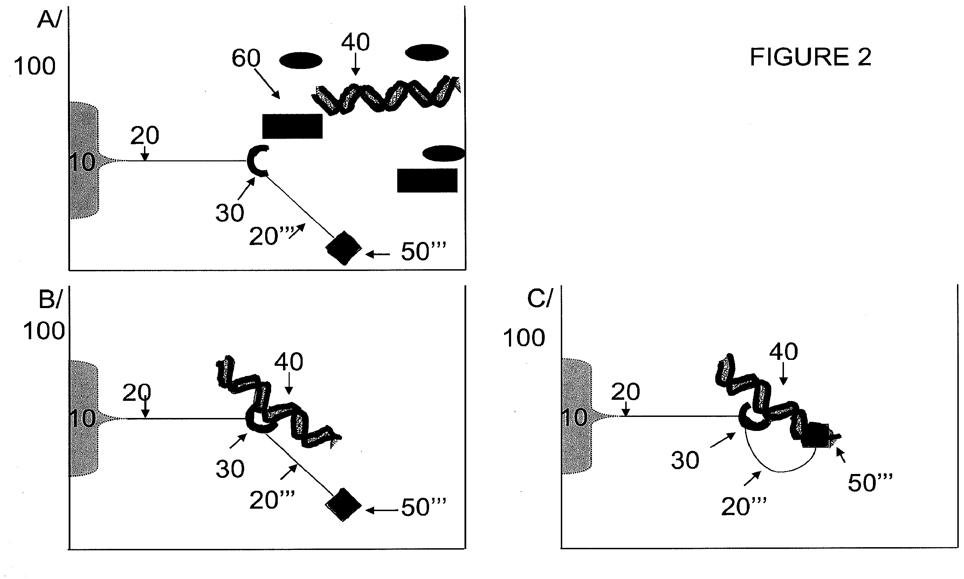

Figure 2 panel A shows a schematic of a general simplified method using a labeled capture system embodiment of the invention. A reaction vessel **100** contains a support **10,** linked to a first capture system **30** through a linker **20.** The support can be, for example, the inner vessel wall or a magnetic particle. In panel B, the capture system discriminately sequesters the target nucleic acid **40** from a crude sample **60,** containing impurities. In Panel C, the label **50'"** linked to the capture system through a linker **20"'** interacts with the conjugate formed by capture system **30** and the target nucleic acid **40.**

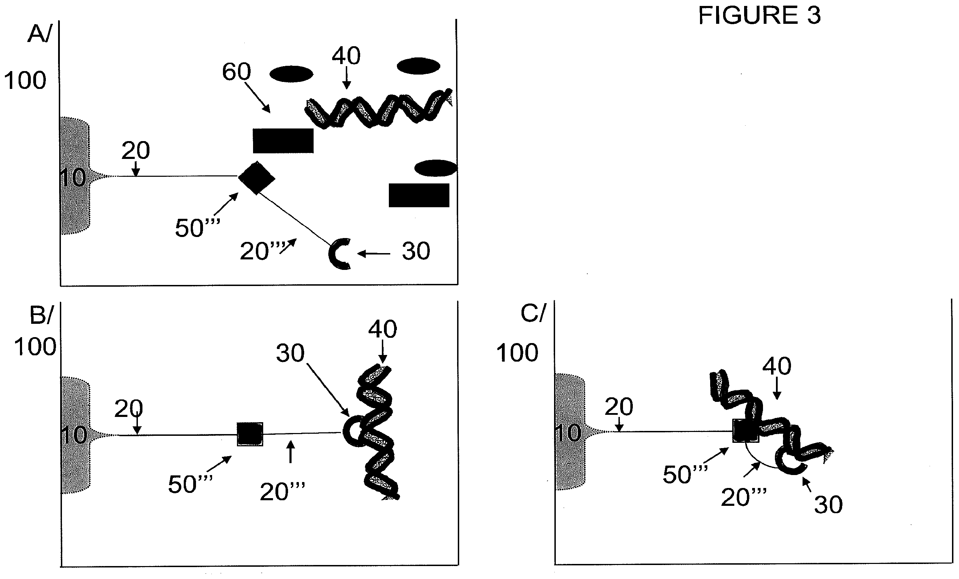

Figure 3 shows a schematic of a general simplified method using a labeled capture system embodiment of the invention. In Panel A, a reaction vessel **100,** contains a support **10,** linked to a label **50'"** through a linker **20.** In Panel B, the capture system **30,** linked to the label **50'"** through a linker **20'",** discriminately sequesters the target nucleic acid **40** from a crude sample **60,** containing impurities. The support can be, for example, the inner vessel wall or a magnetic particle. In Panel C, the label **50'"** interacts with the conjugate formed by the capture system **30** and the target nucleic acid **40.** The components of the three assays are discussed below.

### Target Nucleic Acid

The process of the invention begins with providing a sample **60** containing the target nucleic acid **40,** which is to be isolated and quantified. In some embodiments, the sample **60** is crude blood, plasma, serum, urine or cerebrospinal fluid. In certain embodiments, the sample is a cell lysate.

In some embodiments, samples may be obtained from biological samples such as blood or other bodily fluids, pursuant to a purification, concentration, amplification or dilution process. In some embodiments, samples may be man-made, such as a media effluent from an industrial process. In other embodiments, the sample is a mix of natural and man-made fluids. In various embodiments, samples may be beverages, perfumes, food, or any type of other fluid that could contain free nucleic acids.

In some embodiments, the sample **60** contains intact cells, as well as extracellular nucleic acids. The present invention serves to quantify the extracellular nucleic acids present in a sample and does not quantify the nucleic acids present inside intact cells, should cells also be present within a sample. When the sample is a cell lysate, the invention can quantify double stranded nucleic acids, single stranded nucleic acids, or both types of nucleic acids. In samples where specific kinds of nucleic acids are isolated from a pool of nucleic acids (*e.g.*, mRNA molecules with DNA and ribosomal RNA background), capture systems (discussed *infra*) are designed to sequester the appropriate type of nucleic acid.

In some embodiments, the target nucleic acid **40** is cell free DNA and is present in a blood, plasma, serum or other biological fluid sample. Alternatively, the target nucleic acid **40** can be single stranded nucleic acid, such as mRNA. In certain embodiments, the target is nuclear DNA present in a cell lysate.

In some embodiments, the target nucleic acid **40** is extracted and/or purified, and/or amplified nucleic acid. In embodiments, the nucleic acids are not labeled with a readable label.

### Capture system

In some embodiments, a method provides contacting the target nucleic acid in the sample with at least one nucleic acid interactor to form a conjugate; and sequestering the conjugate by reacting the conjugate with a capture molecule bound to a support. A "capture system" or "capture molecule," as used herein, is a compound or set of compounds used to bind and/or sequester the target nucleic acid. The present invention makes use of at least a first capture system, to sequester the target nucleic acid (herein, "capture molecule 1," "capture system 1" or "first capture system"). In the sandwich method, a second capture system is employed (herein, "capture molecule 2", "capture system 2" or "second capture system") to further bind the sequestered nucleic acid. Capture system 2 is used for the sandwich assay embodiment and is labeled with a readable molecule directly or through a linker. In some embodiments, the second capture system is linked to a molecule that may react with a binding partner linked to a label directly or through a linker.

In embodiments, a first capture system for a target nucleic acid comprises a support, a linker, and a capture molecule that binds to nucleic acids. In embodiments, a first capture system further comprises a readable label or signal generating element. In embodiments, the readable label or signal generating element is bound to the capture molecule and/or the linker.

In other embodiments, a second capture system comprises a capture molecule that binds to nucleic acids, a linker, and a signal generating element.

In some embodiments, a linker is selected that minimizes steric hindrance and chemical interaction with the interactors.

In embodiments, the sample **60** is added to a reaction vessel **100** where the isolation and quantification reactions are performed. In embodiments, the inner walls of the vessel, or a portion thereof, can act as a support **10** for linking a first nucleic acid capture system. In some embodiments, the support **10** is a portion of a glass or quartz microscope slide, and can be a well fabricated in the slide or a well in a plate. In various embodiments, the support **10** is a concentratable particle, which can include a bead/particle, for example, a magnetic particle. In various particle embodiments, the particle is covalently attached to the first capture system. In some embodiments, the particle (immobilized or support) is pre-derivatized with the first capture system **30.**

The derivatization can occur through a linker **20,** which can be a direct covalent bond between the capture system **30** and the support **10.** The linker can also be a single type of molecule or a plurality of different molecules. The linker may contain a specific binding pair (*e.g.*, avidin-biotin, antibody-antigen, enzyme-substrate). In a specific binding pair embodiment, one of the specific pair members is directly bound to the particle and will bind the capture system, by binding or interacting with the partner molecule, which is in turn bound to a nucleic acid interactor *(i.e.,* capture system **30**).

Depending on what molecule for the linker **20** is used, it will be apparent to one of ordinary skill in the art as to how to attach the linker to the substrate. For example, a detailed discussion on attaching biotin to glass substrates is provided in U.S. Patent No. 5,919,523.

The target nucleic acid is isolated from the sample with the use of a first capture system. The first capture system **30** is attached to the support via a linker. The first capture system, in certain embodiments, discriminately isolates double stranded nucleic acids from a sample that contains other impurities and/or single stranded nucleic acids.

In some embodiments, a capture system (first or second capture system) can include as a capture molecule a single stranded or a set of single stranded non sequence-specific nucleic acid (such as for example poly T to bind up poly A of mRNA or poly GC, if there is repetitive DNA -such as CpG islands) intended to form a double stranded nucleic acid or triple stranded nucleic acid with the target, a nucleic acid interactor, and a nucleic acid binding molecule such as a small molecule or a transcription factor, or a chromosomal protein or a combination of them. In certain embodiments, the capture system may be labeled, directly or indirectly, or not labeled. In various embodiments, the capture system may become readable once linked to the conjugate or the sequestered conjugate. In certain embodiments, nucleic acid primers or nucleic acids having sequences complementary to the target nucleic acid (such that the recognition is sequence-specific) are excluded from the capture system.

In various embodiments, the capture system binds the target nucleic acid by non-covalent interactions. Some non-covalent interactions that can be employed are hydrogen bonding, cation- π, π - π interactions, ionic pairing, hydrophobic interaction, dipole-dipole, dipole-induced-dipole, charge-dipole and van Der Waals interactions. In certain embodiments, the capture system comprises antibodies which bind epitopes common to most or all double stranded nucleic acids. For example, antibodies generated in patients with various autoimmune diseases can be harnessed. See Tzioufas AG, Manoussakis MN, Drosos AA et al. "Enzyme immunoassays for the detection of IgG and IgM anti-dsDNA antibodies: clinical significance and specificity." Clin Exp Rheumatol. 1987; 5:247-253.

In the present invention, a nucleic acid interactor or in a preferred embodiment, an intercalator is used as a capture molecule **30.** The interactor can be bound to a specific binding pair member wherein the other member is covalently attached to the support 10, for example, a paramagnetic particle. The sample **60** is added to the reaction mixture and mixed with the interactor-derivatized particle. The particle can then be sequestered from the sample by applying a magnetic field to the vessel and decanting the liquid phase.

A "nucleic acid interactor" or "nucleic acid interacting molecule", as used herein, refers to a molecule that binds to nucleic acids with specificity to ensure subsequent steps of the process. The interactor is therefore capable of capturing and/or isolating (*i.e.,* sequestering) a target nucleic acid from a sample. Nucleic acid interactors include, without limitation, nucleic acid intercalating agents (also referred to as intercalators), as well as minor and major groove binders, small molecules which bind nucleic acid in a non-sequence-specific manner, non-sequence-specific nucleic acids, fusion molecules resulting from a combination of several molecules or from a set of molecules and single stranded nucleic acids (*e.g.,* peptide-nucleic acid chimera) and antibodies, or any type of other combination. These molecules are used as the capture system to initially sequester the target nucleic acid. In some embodiments of the invention, a nucleic acid interactor can act as both an intercalating agent and a minor or major groove binder. In embodiment, the nucleic acid interactor does not permeate a cell.

In certain embodiments, the support **10** is covalently bound to the first capture system **30.** In these embodiments, the covalent bond between the support and capture system is the linker **20.** In some embodiments, the support is covalently bound to a linker molecule which binds the capture system either covalently or non-covalently.

In embodiments, nucleic acid interactors are harnessed as the main component of the first capture system **30** and/or second capture system **30'.** In some embodiments, the capture molecule in the first capture system and the second capture system are different from one another. In other embodiments, the capture molecule in the first capture system and the second capture system are the same. In some cases, when two or more different nucleic acid interactors are used only one of the nucleic acid interactors is bound to a support. In yet other cases, when two or more nucleic acid interactors are used one of the nucleic acid interactors is bound to a support, a different nucleic acid interactor is bound to a label. In some embodiments, two or more nucleic acid interactors are selected to have similar spectral properties. In some other embodiments, two or more nucleic acid interactors are selected to bind to different types of nucleic acids or bind at a different structural feature of a nucleic acid.

The interactors, such as DNA intercalators, can be bound to a substrate (*e.g.,* beads) through a linker **20** or **20',** which can be a linking molecule or simply a covalent bond. Suitable linkers for intercalator embodiments are known; some are, for example, described in U.S. Patent No. 4,921,805.

A "linker," or "linking moiety," for use with the present invention, is any molecule, set of molecules (*e.g.,* binding partners, such as biotin/streptavidin, antibody/antigen), or chemical bond, which has served to connect either (1) the support and the first capture molecule, (2) each of the first and second capture molecules with the label (sandwich assay embodiments), (3) a competing nucleic acid and a label (competitive method embodiments), or (4) a label and a support (one labeled capture system method). In some embodiments, the linker is a peptide, a single stranded nucleic acid, a carbon chain, or simply a covalent bond. In other embodiments, the linker is an antibody or nucleic acid binding molecule. In some embodiments, the linker is composed of a plurality of molecules.

In some embodiments, the capture molecule and the solid support **10** are first modified in such a way that they can be joined together through an amide, carbamate, urea, ether, thioether, amine or other linkage commonly used for immobilization (Affinity Chromatography. Hoffman-Osterhof, ed.; Pergamon Press, 1978; Affinity Chromatography, publication of Pharmacia Fine Chemicals). For example, if the solid support **10** is modified to contain a carboxy group, then the intercalator or interactor may be modified to contain a reactive amine through addition of a spermine or diaminoalkane molecule. Using coupling agents such as dicyclohexylcarbodiimide, the support and the intercalator can be joined together through an amide bond. Alternatively, the carboxyl group can be converted to a reactive ester (such as N-hydroxysuccinimide ester) which will then react with the amine. Conversely, the support can be modified with an amine and the nucleic acid interactor can be modified to contain a carboxyl group and the two moieties coupled together as described above.

The linkers **20, 20'** and **20",** can be covalent bonds, as described above, or can be a molecule or plurality of molecules, such as specific binding pairs. The linker molecule may be charged or uncharged, and in certain embodiments, comprises methylene groups joined together with amide bonds (see, *e.g*., U.S. Patent No. 4,921,805).

The linkers **20, 20', 20"** and **20'"** of the present invention, must be bifunctional in the sense that it can bind to both the support and first capture system (**20**) or to both the second capture system and the label (**20'**) or to both the competing nucleic acid and the label (**20"**) or to both the first capture system and the label (**20"'**).

In certain embodiments, the linker is the shortest path connecting the two linkable entities (*i.e.,* capture system 1 and support (**20**), capture system 2 and label (**20'**), competing nucleic acid and label (**20"**), capture system 1 and label (**20"'**)). A linker, in some embodiments, is a chain of atoms or a branched chain of atoms. Chains can be saturated as well as unsaturated. The linker may also be a ring structure with or without conjugated bonds. It can be a molecule that consists of alkyl, alkenyl, alkynyl or aryl groups, as well as any combination of these groups. The linker can also comprise a fluorescent substrate, for use in FRET assays.

In some embodiments, the linker **20** is substituted by any chemical group with one or more atoms selected from the group consisting of C, H, O, S, N, P, Se, Ge, Sn and Pb. For example, the linker **20** may comprise a chain of "m" atoms selected from the group consisting of C, O, S, N, P, Se, Ge, Sn, Pb, wherein one end of the chain is connected to the first entity (*i.e.,* support, capture system 2, or competing nucleic acid) and the other end of the chain is connected to the second entity (*i.e.,* capture system 1 or label). In various illustrative embodiments, the linker **20** can be a bond, a specific binding pair, a peptide, a pseudopeptide, a nucleotide or a pseudonucleotide, a polysaccharide, and can either be branched or linear. The linker molecule may or may not be substituted.

The total length of the linker **20** and its chemical structure are adjusted depending on the size and the chemical structures of the nucleic acid interacting molecule (*e.g.,* intercalating agent) and linking entities. The optimum adjustment of these parameters can be achieved (based in part on the type of linker) by molecular modelling by using an empirical force field like CHARMM (Brooks, B. R., Bruccoleri, R. E., Olafson, B. D., States, D. J., Swaminathan, S., and Karplus, M. (1983) J. Comput. Chem. 4, 187-212) defining solvent mimicking the *in vitro* environment, taking into account concentration of salt and buffer, and the pH of the solution. Empirical testing can also be employed to determine optimal linker properties.

A preferred linker for a specific couple of linking entities can be designed to: (1) be energetically stable in a range of pH and temperature values compatible with the reaction performed for DNA quantification (*e.g.,* the luminescent reaction or magnetic detection) and avoid degradation in a range of pH and temperature values compatible with the biochemical reaction performed for DNA quantification.

In preferred embodiments, the linker **20** is energetically stable and nondegradable for at least a period of time from the beginning of the assay (capture of target) through completion of the time needed until performing the readout reaction, as well as when subjected to the physical conditions and environment (e.g., pressure, hygrometry, vibrations, pollution, temperature, noise, light, agitation, etc.) needed to carry out the reaction. Linker stability should also not be affected by dilution.

The linker **20** can be inert with the rest of the constituents of the reagents' medium and should be soluble in the medium where the assay of the present invention is conducted.

In addition, the linker **20,** in preferred embodiments, does not adopt a conformation that sterically interferes with the readout reaction or with the nucleic acid capture. The linker **20** also avoids the alteration of the folding and/or the chemical structure of the support **10** and of the nucleic acid interactor (**e.g.,** intercalating agent) by, for example, electron delocalization, radical generation, electronic modifications leading to a loss of intercalating, interacting, magnetic or optic or luminescent properties, or a loss of integrity of the support leading to a release of the linker/intercalating agent couple from the surface of this sited support.

In some embodiments, the linker **20** is compatible for additional conjugated chemical reactions in the medium (*e.g.,* binding an antibody specific for one member of the support/linker/intercalating agent/target molecule complex, without altering the structure and stability of the complex, except if this effect is needed for the correct achievement of the reaction).

Importantly, the linker **20** should have a chemical structure compatible with the attachment with one or more nucleic acid interacting molecules and support.

In some embodiments, a first and/or second capture system comprises a readable label. In some embodiments, the readable label is bound to the linker and in others, to the capture molecule. A "label", as used in the present invention, is any moiety which can be used in the readout reaction and which binds the target nucleic acid or competing nucleic acid, through a linker (competitive assay) or through an additional capture system and linker (sandwich assay). Multiple labels may be employed in order to identify different types of nucleic acids, e.g. double stranded DNA, mRNA, and the like.

In some embodiments, the label may be linked, directly or indirectly, to the first capture system and link itself to the support, directly or indirectly. In this case, the capture system binds the nucleic acid, in a non-sequence-specific manner, and there is no need for a second step to bind the label, already linked, via the capture system, to the conjugate. In certain embodiments, the entity formed by the capture system and the label may become readable once the capture system binds to the target, or once the label links itself to the conjugate (see Fig 1C).

In some embodiments, the label may be linked, directly or indirectly, to the support. It may be linked, directly or indirectly, to the capture system. In this case the capture system binds the nucleic acid, in a non-sequence-specific manner, and there is no need for a second step to bind the label, already linked, via the capture system, to the conjugate. In certain embodiments the entity formed by the capture system and the label may become readable once capture system binds to the target, or once the label links itself to the conjugate (see Fig 1D).

In certain embodiments, in which luminescence is used as the readout method, the label will be the moiety from which light is emitted. In some embodiments the label can be acridinium, ruthenium, Europium, XL665 or dioxetane.

In various embodiments, the label is a particle detectable in a magnetic field (e.g. magnetic, ferromagnetic paramagnetic or superparamagnetic beads (Dynabeads)).

The signal emitted by the label can be optic (UV, Visible, IR absorption), fluorescent, luminescent or magnetic, it may be detected without limitations by NMR, circular dichroism or Raman spectroscopy.

In certain embodiments, the label is an enzyme that modifies its substrate so as to be detected by any means cited *supra,* including luminescence.

A "labeled conjugate", as used herein, is a conjugate, sequestered or otherwise, further bound in some manner to a label. A "sequestered labeled conjugate," refers to a labeled conjugate further bound to a support.

"Essentially exclusively labeling a sequestered conjugate", as used herein, means binding a label to a sequestered conjugate, directly or indirectly. Non-specific binding of the label to components other than sequestered conjugates may occur, but the signal generated from these interactions can be subtracted from the total signal and, therefore, false positive signals will not be counted. The non-specific labeling can be quantified by a calibration step before the method is carried out. In some embodiments, the ratio of the label bound to a sequestered conjugate as compared to other components in the reaction is 1000:1, 500:1, 100:1 or 10:1. In some embodiments, the ratio is 5:1 or 2:1.

In the present invention, when a conjugate is "essentially exclusively labeled," the labeling starts in solution, *i.e*., the support is not labeled with the readable molecule prior to the addition of target nucleic acid (the support is not "prelabeled"). The earliest the label can associate *(i.e.,* indirectly bind, *see* Fig. 1A) with the support is at the time the target nucleic acid binds the first capture system. The first capture system is the reaction component that will either be bound to the support originally *(see* Fig. 1A), or will bind the support after introduction into the reaction. The label cannot indirectly bind the support without the first capture system and the nucleic acid molecule *(see* Fig. 1A).

The first capture system **30** can be any molecule or molecules, attached to the support **10** via a linker **20,** and serves to isolate the desired nucleic acid from a crude sample *(i.e.,* a sample that contains undesirable nucleic acids and/or non-nucleic acid components). In a preferred embodiment, the first capture system comprises a nucleic acid intercalating agent.

Based on the capture system **30** chosen, it will be apparent to one of ordinary skill in the art, how to immobilize the capture system or in any event attach it to the support.

In some embodiments, the support **10** will be prederivatized with the capture system **30.** In other embodiments, the support **10** will sequester or bind the capture system **30** when the user combines the two in a reaction, *e.g.,* by use of specific binding pairs.

In various embodiments, the target nucleic acid **40** to be detected is a single stranded molecule. When this is the case, the first capture system **30** can be comprised of a molecule that binds, in a non-sequence-specific manner, the target based on secondary structure. In other embodiments small molecules which bind universal stretches of nucleic acids can be used to sequester target nucleic acids from the sample. In the small molecule embodiments, multiple small molecules can be harnessed, in order to bind all possible nucleotide stretches. This can be accomplished by derivatizing the support with a library of small molecules. Although single stranded nucleic acids can be isolated and quantified from a crude sample by the methods presented herein, a preferred target is a double stranded nucleic acid. When the target is double stranded, a preferred capture system 30 is a set of nucleic acid intercalators. The present inventors have shown that the two intercalators help eliminate or significantly reduce measurement dependency on target size and/or target sequence.

In various embodiments, one or a set of single stranded non sequence-specific nucleic acids, can be used as a portion of the first capture system **30** and can sequester, for example, target nucleic acid **40** from the sample **60** to form a double helix, or target double stranded nucleic acid **40** to form a triple helix. In these embodiments, a nucleic acid intercalator is not necessary for use in the first capture system **30,** although a combination of capture molecules (*e.g.,* single stranded nucleic acid & intercalating agent), or fusion molecules, can be used in combination as first capture systems, in the same reaction.

In certain embodiments, wherein the target nucleic acid **40** is single stranded, double stranded molecules are made by adding single stranded nucleic acids to the reaction before sequestration of the single stranded target nucleic acid **40** by the first capture system **30.** In these embodiments, careful consideration is paid to the sequences of the additional single stranded nucleic acids, as the formation of non-specific double helices will lead to false positives in the quantification step. In some embodiments, the single stranded nucleic acid molecules added to the sample will be part of a fusion molecule, resulting from a combination of several molecules or molecules and single stranded nucleic acid (*e.g.,* peptide-nucleic acid chimera). In these embodiments, the fusion molecule can be considered a portion of the first capture system **30.** In other embodiments, a nucleic acid intercalator is used as the capture system **30** to sequester the newly formed double stranded molecules from solution.

In other embodiments, the single stranded, or a set of single stranded non sequence-specific nucleic acid complements, can be used as a capture system **30.**

In some embodiments, the first capture molecule **30** can be an antibody, a portion thereof, or a protein other than an antibody, which will recognize the peptide portion of the fusion molecule (described *supra*). Alternatively, a capture system **30** can be comprised of a protein that can recognize stretches of DNA, *e.g.,* a transcription factor. In some embodiments, the first capture system **30** (and/or second capture system **30',** depending on whether a sandwich assay is employed) is a nucleic acid binding molecule, such as a transcription factor or histone protein, which will specifically bind a target nucleic acid **40.** Small (natural or synthetic) molecules can also be used as a capture system **30.** For example, molecules disclosed in DNA and RNA Binders, From Small Molecules to Drugs, Martine Demeunynck, Wiley-VCH (2003) and Nelson et al. Mutat Res., V. 623, pp. 24-40 (2007), can be used as capture molecules of either the first **30** or second **30'** capture system.

In certain embodiments, the first **(30)** or second **(30')** capture system is an antibody or a library of antibodies specific for epitopes present on the target nucleic acid **40.** The second capture system **30'** can be an antibody, a plurality of antibodies (*e.g.,* library of antibodies specific for epitopes present on the target nucleic acid), or epitopes spanning the first capture system **30** and the target nucleic acid **40.**

In other embodiments, the capture system **30** comprises one or a plurality of polymers which can bind either nucleic acids or intercalating agents, or both, such as the ones disclosed in U.S. Published Application No. 2004/0157217.

The first **30** and second **30'** capture system can make use of non-covalent interactions to bind the free target nucleic acid or sequestered nucleic acid. Some non-covalent interactions that can be employed are hydrogen bonding, cation- π, π - π interactions, ionic pairing, hydrophobic interaction, dipole-dipole, dipole-induced-dipole, charge-dipole and van Der Waals interactions.

The capture system **30** or **30'** may comprise a molecule or several molecules. It reacts with nucleic acid by establishing covalent or non-covalent interactions with the target nucleic acid **40,** or in the case of the second capture system **30',** interactions can be between the capture system and epitopes spanning the first capture system **30** and target nucleic acid **40.** Specific capture systems that may be employed include, but are not limited to intercalating agents, cyanine dimers, cell impermeant cyanine monomers, minor or major groove linkers, orange acridin, 7-AAD, LDS 751, and hydroxystilbamidin. Such agents are described in Chapter 8 of The Handbook - A Guide to Fluorescent Probes and Labeling Technologies, 11th Edition, published by Molecular Probes).

In various embodiments, an intercalating agent for use as a capture system **30,** or a component of a capture system, can be, without limitation, for example, echinomycin, ethidium, ethidium bromide, propidium bromide, methidium, acridine, aminoacridine, quinacrin, acridine orange and derivatives thereof, psoralen, proflavin, ellipticine, actinomycin D, daunomycin, malachite green, phenyl neutral red, mitomycin C, Hoechst33342, Hoechst33258, aclarubicin, DAPI, SYBR, Picogreen, Adriamycin, pirarubicin, actinomycin, tris (phenanthroline) zinc salt, tris (phenanthroline) ruthenium salt, tris (phenantroline) cobalt salt, di (phenanthroline) zinc salt, di (phenanthroline) ruthenium salt, di (phenanthroline) cobalt salt, bipyridine platinum salt, terpyridine platinum salt, phenanthroline platinum salt tris (bipyridyl) zinc salt, tris (bipyridyl) ruthenium salt, tris (bipyridyl) cobalt salt di (bipyridyl) zinc salt, di (bipyridyl) ruthenium salt, di (bipyridyl) cobalt salt, luzopeptin, triostin A, oxazole yellow, thiazole orange TOTO, eth D., TOTA B, YOYO and derivatives and dimers of the foregoing.

In some embodiments, a minor groove binder is used as a nucleic acid interactor (and as a capture system **30**). The minor groove binder can be, without limitation, for example, Hoechst 33258, netropsin, distamycin, plicamycin, CDPI₁₋₃, lexitropsin, mithramycin, chromomycin A₃, olivomycin, anthramycin, sibriromycin, pentamidine, stilbamidine, berenil, or any other minor groove binder.

Still, in other embodiments, a capture system **30** can employ an interactor that acts as both a nucleic acid intercalator and a (minor or major) groove binder. For the purposes of the assay, it is irrelevant as to how the interactor sequesters the target nucleic acid **40** from the remaining sample components, so long as the target nucleic acid **40** is sequestered. Heterogeneous capture systems that use both an intercalator and groove binder can also be used as capture system 1 or 2 (30 or 30' respectively).

In other embodiments, the first capture system **30** selectively isolates target single stranded nucleic acid **40** from a sample **60** that may have double stranded nucleic acid.

In some embodiments, reacting material may be transferred, at any step, from one vessel to another, by automated process.

### Sandwich assays

The sandwich assay embodiment makes use of a second capture system **30'.** Once target nucleic acids **40** are sequestered from the crude sample **60**, the second part of the reaction may occur in the same vessel or after automatic transfer by the machine, in another one. This can be done immediately or after a wash or buffer exchange step, to remove sample impurities. Wash steps can be automated. Then, the second capture system **30'** is added to the reaction. When using particles as the support **10** for the first capture system, the sample **60** (containing the target nucleic acid **40**) is added to the reaction and mixed with the particles. The particles (now bound to the target molecules) are sequestered by applying a magnetic field. At this point, a wash/supernatant removal step can be employed. The magnetic field is then discontinued and a second capture system **30',** labeled with a readable molecule **50,** is added to the reaction, optionally after a wash step. After incubation, particles are sequestered. After the wash step, the reading method is activated in the same vessel or after automatic transfer by the machine, in another one.

Alternatively, the two capture systems (**30** and **30'**) may be added to the reaction simultaneously. After incubation with both capture systems, excess capture systems are removed from the reaction with a wash step. After the wash step, the reading method is activated in the same vessel or after automatic transfer by the machine, in another one.

If a capture system is bound to the wall of a reaction well or vessel, automated or manual wash steps can be employed after nucleic acid sequestration. These steps serve to remove impurities from the original sample.

Once the target nucleic acid **40** is isolated/sequestered from the sample **60,** the user can proceed with detection (and quantification) of the nucleic acid. In the case of the sandwich assay, a second capture system **30'** (which is labeled) is added to the reaction. The second capture system **30'** comprises a nucleic acid interactor that will bind to the double, the triple stranded, or to a free part of the single stranded sequestered nucleic acid, or with the structure resulting from the interaction between capture system 1 and nucleic acid. The two capture systems may be added simultaneously or in a serial manner.

The sequestered nucleic acid is essentially exclusively labeled with the readable molecule, and nonspecific labeling of other reaction components is factored into the end signal. This is accomplished through an initial calibration step.

The calibration step is carried out by measuring known amounts of nucleic acids, subjected to the process of the present invention. Increasing known amounts of nucleic acids are measured, in order to generate a linear relationship between the detected signal (*e.g.,* light emitted) and nucleic acid molecules initially present (also known as a *standard curve*). The linear relationship has both an upper and lower limit, which establish the dynamic range of the assay. The measured signal is proportional to the amount of nucleic acid molecules initially present. Therefore, when an unknown target nucleic acid **40** is subjected to the process disclosed herein, the number of target nucleic acid molecules initially present can be extrapolated from the standard curve, by determining where along the curve the generated signal falls.

The amount of target nucleic acid molecules present in the sample is then compared to a value that corresponds to the amount of nucleic acids in a sample from a subject that does not have a particular disease or disorder. For example, if a sample analyzed from a subject suspected of having cancer has an amount of target nucleic acid that is at least two fold greater, at least 4 fold greater, at least 5 fold greater, or at least 10 to 100 fold greater than that from a sample from a subject without cancer is indicative of a pathological condition. In some embodiments, an amount of cell free nucleic acid in the sample of at least about 5 ng/ml or at least 10 ng/ml is indicative of a pathological state.

In embodiments, a result indicative of a pathological state is then communicated to a health care provider. The health care provider will order or administer one or more additional diagnostic tests to detect the pathology. Such tests can include, tests for one or more cancers including biomarker tests and biopsies, tests for cardiac injury and/or liver injury by detecting elevated cardiac or liver enzymes, and the like.

The second capture system 30' (*e.g.,* a DNA binding protein, interactor, antibody) is covalently or non-covalently bound to the label 50 via a linker 20'. In various embodiments, the second capture system 30' may become activated and then readable after interaction with the conjugate.

Nucleic acid can be detected by methods known to those of ordinary skill in the art. Luminescent labels can be read off by harnessing fluorescence resonance energy transfer (FRET), electronic or electrochemical excitation (to excite a fluorescent label), or by chemiluminescence or bioluminescence reactions. In some embodiments, the labeled capture system will bind subpopulations of target nucleic acid **40** and are linked to distinct labels to allow for multiplex detection.

Examples of luminescent reactions amenable for use with the present invention are, without limitation, luciferase-mediated oxidation, acridium ester with hydrogen peroxide, alkaline phosphatase with dioxetane, dioxetane with *β* - galactosidase, horseradish peroxidase with luminol and gold mixed with luminol, or electrochemical excitation of ruthenium and tripropylamine.

In some embodiments, luminescent detection is the detection of a fluorescent substrate. The second capture system **30'** is bound via a linker to a fluorescent dye or molecule (**50**); it may also be fluorescent by itself once linked. Once excess capture systems **30'** are removed, the fluorescent molecules are excited at the appropriate wavelength and fluorescent intensity is read out. The amount of luminescence or fluorescence detected is proportional to the amount of target nucleic acid **40** in the sample **60.**

In some sandwich assay embodiments, wherein the first capture system **30** forms a triple helix with the target DNA, the second capture system **30'** can comprise a triple helix intercalator or interactor. Some examples of triple helix intercalators that can be used are bis-4-aminoquinolines and extended ethidium bromide analogues. Examples of ethidium bromide analogues are given in Wilson et al. Biochemistry, V. 32, pp. 10614-10621 (1993). The embodiment where the first capture system **30** is a single stranded nucleic acid which binds double stranded target molecules can employ this type of second capture system **30'.**

The second capture system **30'** can be covalently bound to the readable substrate or by way of a linker molecule **20',** or just a covalent bond. In other embodiments, the linker **20'** between the second capture system and luminescent substrate contains a specific binding pair, which interacts non-covalently (*e.g.,* biotin-avidin complex). The linker must be long enough to allow for the interactor to bind the double or triple stranded nucleic acid. When the readable substrate is bound directly to the interactor, careful consideration is paid to the binding site of the substrate on the interactor, so as to preclude steric blocking of substrate binding.

Unbound second capture systems **30'** are removed in wash/supernatant removal steps. Particles can be sequestered and the unbound capture systems removed. Alternatively, manual or automated pipetting steps are employed to wash the walls of reaction vessels. The wash/supernatant removal step ensures accurate quantification, *e.g.,* by removing components that can interfere with the readout reaction, by removing components that contribute to background noise or that non-specifically bind to reaction components.

Once the unbound labeled capture systems are washed away, the second step of the reaction may occur either in the same vessel or another one after automatic transfer by the machine.

In some embodiments, the readout reaction will use luminescence. Depending on the luminescent reaction chosen, it will be apparent to one of ordinary skill in the art how long to carry out the reaction, and at what temperature, to ensure accurate and reliable quantification, as parameters for carrying out luminescent reactions are known. If conditions are not optimal, empirical optimization can be carried out to determine reaction parameters. The two capture systems may be added simultaneously. After incubation, unbound material is washed away and detection may occur.

In other embodiments, chemical or physical detection of the label **50** is done. It will be apparent to one of ordinary skill in the art how long to carry out the reaction, and at what temperature, to ensure accurate and reliable quantification, as parameters for carrying out detection are known. If conditions are not optimal, empirical optimization can be carried out to determine reaction parameters. The two capture systems (**30** and **30'**) may be added simultaneously. After incubation, unbound material is washed away and detection may occur.

### Competitive method

In the competitive method, a known concentration of labeled nucleic acid **40'** is added to the reaction with the target nucleic acid **40.** The label **50"** is linked to the nucleic acid **40'** via a linker **20",** described *supra.* The labeled nucleic acid competes with the unlabeled target nucleic acid for binding to the first capture system **30.** After an incubation time, the excess of labeled nucleic acid fragments are removed in concert with an optional wash step and the readout reaction is started, either in the same vessel or another one after automatic transfer by the machine. Signal is then measured and the intensity is inversely proportional to nucleic acid concentration in the sample **60.**

In some embodiments, readable detection is the detection of a fluorescent substrate. The second capture system **30'** is bound via a linker **20'** to a fluorescent dye or molecule (**50**). Once excess of labeled nucleic acids **40'** is removed, the fluorescent molecules are excited at the appropriate wavelength and fluorescent intensity is read out, either in the same vessel or another one after automatic transfer by the machine. The amount of fluorescence detected is inversely proportional to the amount of target in the sample **60.**

In various competitive and sandwich assay embodiments, a reagent which quenches a given luminescence reaction can be added after luminescence detection in order to allow for subsequent wells to be assayed without refractive or reflective cross-talk between wells. Such reagents are disclosed in U.S. Patent No. 5,744,320. These reagents can be used for both sandwich and competitive assays.

The amount of target nucleic acid molecules present in the sample is then compared to that a value that corresponds to the amount of nucleic acids in sample from a subject that does not have a particular disease. For example, if a sample analyzed from a subject suspected of having cancer has an amount of target nucleic acid molecules that is at least two fold greater, at least 4 fold greater, at least 5 fold greater, or at least 10 to 100 fold greater than that from a sample from a subject without canceris indicative of a pathological condition. In embodiments, an amount of cell free nucleic acid of at least about 5 ng/ml or at least about 10ng/ml is indicative of a pathological state.

### Single step capture system method

In some embodiments, first capture system **30** may contain a nucleic acid interacting molecule or a set of molecules and a label **50.** Once nucleic acid interaction occurred, label may react with the sequestered conjugate and may become active. Once impurities are removed by a washing step, the signal is then measured with the appropriate method, either in the same vessel or another one after automatic transfer by the machine. Intensity is proportional to nucleic acid concentration in the sample **60.**

In some embodiments, readable detection is the detection of a fluorescent substrate. The capture system **30** is bound via a linker **20** to a fluorescent dye or molecule (**50**). Once impurities are removed by a washing step, the fluorescent molecules are excited at the appropriate wavelength and fluorescent intensity is read out, either in the same vessel or another one after automatic transfer by the machine.

The capture system **30** is linked to the support **10** via a linker **20,** directly or indirectly. In some embodiments capture system **30** is linked to the wall of the vessel, directly or indirectly.

The label **50** is linked to the capture system **30** via a linker **20,** directly or indirectly. It may react with the structure resulting of interaction between capture system and nucleic acid, or with a free part of the sequestered nucleic acid.

In certain embodiments, if the label **50** is linked to the capture system **30** via a binding pair (*e.g.,* biotin/streptavidin...) it may be added simultaneously with the sample or in a second step after washing of the impurities. This further addition may occur in the same vessel or another one after automatic transfer by the machine. It is then possible to add a new washing step to remove excess of unbound label.

In certain embodiments, the label **50** may be a molecule that acquires power, or that becomes readable, only when reacting with nucleic acid.

In some embodiments the capture system **30** may act as the label **50.** It may interact with the target nucleic acid **40** and then, after acquiring power, be detected with an appropriate method.

The amount of target nucleic acid molecules present in the sample is then compared to a value that corresponds to an amount of nucleic acids in a sample from a subject that does not have a particular disease. For example, if a sample analyzed from a subject suspected of having cancer has a number of target nucleic acid molecules that is at least two fold greater, at least 4 fold greater, at least 5 fold greater, or at least 10 to 100 fold or more greater than that from a sample from a subject without cancer is indicative of a pathological state. In embodiments, an amount of cell free nucleic acid of at least about 5 ng/ml or at least 10 ng/ml is indicative of a pathological state, i.e., a disease or disorder.

### Kits

Another aspect of the disclosure provides kits. In embodiments, a kit for quantifying a target nucleic acid in a sample comprises (i) a first nucleic acid interactor; (ii) a second nucleic acid interactor that is labeled with a readable label and which interactor is different from said first nucleic acid interactor; (iii) a label, optionally bound to the first interactor; and(iv) instructions for use. In embodiments, the kit further comprises control nucleic acid fragments of different sizes and sequences.

In some embodiments, a kit comprises a first capture system as described herein. In embodiments, the first capture system comprises a first capture molecule, such as a nucleic acid interactor bound to a support. In embodiments, the support and/ or the capture molecule are labeled with a readable label as described herein.

In some embodiments, a kit further comprises a stock solution of a control nucleic acid. The control nucleic acid can be diluted and used to generate a standard curve to provide for quantification of a target nucleic acid in a sample. In embodiments, the control nucleic acid is a set of nucleic acids each having a different size, for example from about 200 to about 1000 base pairs and any size in between.

In some embodiments, the kit further comprises instructions for use. In embodiments, the instructions provide for implementation of steps of the methods as described herein. A determination of the result of the number or amount of target nucleic acids in the sample is then compared to that of a control sample and/or to a cutoff value as provided in the kit. In embodiments, a kit may further comprise a control sample from a subject not known to have a pathological condition. In embodiments, a cutoff value indicative of a pathological state is at least about 5 ng/ml or 10ng/ml of nucleic acid.

In embodiments, a kit further comprises a second capture system as described herein. A second capture system comprises a capture molecule, such as a nucleic acid interactor that is labeled with a readable label. The second capture molecule may be the same or different than the first capture molecule. In embodiments, both capture molecules have similar spectral properties.

In some embodiments, a kit further comprises one or more other nucleic acid interactor molecules.

In one embodiment, a kit further comprises a known quantity of a labeled nucleic acid molecule. This known quantity of labeled nucleic acid molecule is used to generate a standard curve and to compete for binding with the unknown target nucleic acid in the sample. In some embodiments, the known quantity of labeled nucleic acid is an isolated double stranded DNA, isolated mRNA, isolated single stranded DNA, isolated miRNA, and combinations thereof. When multiple types of known nucleic acids are utilized, they each may be labeled with a different readable label so that each may be detected in a single sample.

The present invention is further illustrated by reference to the Examples below. However, it should be noted that these Examples, like the embodiments described above, are illustrative and are not to be construed as restricting the enabled scope of the invention in any way.

### Example 1A

One example of a sandwich based assay uses second capture systems **30'** labeled with acridinium. The sample is added to the reaction vessel containing a first capture system **30,** which binds to target nucleic acid **40.** This capture system is linked, via a linker **20,** to a paramagnetic microparticle. The target nucleic acid **40** is incubated with the capture system **30** and the target nucleic acid **40** binds to the capture system, forming a conjugate (*e.g.,* an intercalating complex). After an optional wash/supernatant removal step, a second capture system **30'** is added. This second system is labeled with a chemiluminescent acridinium compound. It binds to nucleic acid/first capture system conjugate. After incubation time, a magnetic field is applied to the vessel and the paramagnetic microparticles are sequestered to a wall of the reaction vessel. The vessel is then washed to remove unbound material, *i.e.,* excess of the labeled second capture system. The light generation reaction is started, after addition of a specific reagent (reagents containing, *e.g.,* hydrogen peroxide, acidic medium, and sodium hydroxide) that chemically excites acridinium. The luminescence is measured by an analyzer. The intensity of luminescence is proportional to nucleic acid concentration in the crude sample. The absolute quantity of nucleic acid can be determined by plotting the results of the experiment on a standard curve generated with known quantities of nucleic acids, taking into consideration sample loss due to handling.

The above example can be further modified as follows: The second capture system **30'** is a biotin-labeled compound. It binds to sequestered target nucleic acid **40.** Avidin labeled with acridinium is added to the reaction along with the second capture system **30'.** Avidin reacts with biotin linked to the intercalating complex. After an incubation time, a magnetic field is applied to the reaction vessel, which serves to sequester the microparticles to a wall of the reaction vessel. The vessel is washed to remove unbound material, *i.e.,* excess of the second capture system **30'** and avidin labeled acridinium. The magnetic field is discontinued and the luminescence reaction is started, after addition of a specific reagent (*e.g.,* hydrogen peroxide, acidic medium, and then sodium hydroxide or Bayer reagent) that triggers a chemical excitation of acridinium.

### Example 1B

The sample **60** along with the first capture system **30** are added to a reaction vessel, either manually or in an automated fashion. The capture system is linked, via a linker **20,** to a paramagnetic microparticle. The linker **20** is labeled itself with a cryptate molecule. The reaction mixture incubates and the nucleic acid binds with the capture system, forming a conjugate. Optionally, a wash step can be employed at this point to remove impurities from the sample **60.** To the previous mixture, the second capture system **30'** is added. This second system is labeled with a XL665-molecule. It binds to nucleic acid/first capture system conjugate. After an incubation time, a magnetic field is applied to the vessel which attracts the microparticles to a wall of the reaction vessel. The vessel is washed to remove unbound material, *i.e.,* excess of the XL665-labeled second capture system. The magnetic field is discontinued and the light generation reaction is started by the reading instrument, by light excitation of the cryptate at 337 nm. Cryptate emits light which excites XL665, which emits light (665 nm). The fluorescence intensity is then measured and quantified by the instrument.

### Example 1C

The reagents in Example 1A can be further modified to include dioxetane phosphate-labeled- or ruthenium labeled-second capture system **30'.** After wash and supernatant removal steps, the reaction of light generation is started, after manual or automated addition of a specific Beckman reagent (when using dioxentane -Lumi-Phos 530, phosphatase alcaline, surfactant fluorescein) or a specific Roche reagent (ruthenium - TPA, oxidizes ruthenium). The luminescence is then measured by the analyzer. The linker **20** can comprise specific binding pair, such as avidin and biotin, one of which will be attached to the microparticle.

### Example ID - Dade Behring reagents

A sample **60** containing target nucleic acid **40** is added, either manually or in an automated fashion, to a vessel containing a first capture system **30,** which binds to the target nucleic acid **40.** This capture system is linked, via a linker **20,** to a chemibead microparticle (*i.e.*, the support). The reaction mixture incubates and the nucleic acid binds with the capture system, forming a conjugate. Optionally at this point, a wash step can be employed to remove any sample impurities. A second capture system **30'** is then added to the reaction vessel. This second system labeled with a specific binding pair member, *e.g.,* biotin. The second capture system **30'** binds to the sequestered nucleic acid, as described *supra.* After an incubation time, a magnetic field is applied in order to sequester the paramagnetic chemibead microparticles to a wall of the reaction vessel. The vessel is washed to remove unbound material, *i.e.*, excess of the labeled second capture system. The magnetic field is removed and label catchers linked to sensibead microparticles are added. The label catcher is the other member of the specific binding pair, *e.g.,* avidin. It binds to the label **50** of the second capture system **30'.** After an incubation time, a magnetic field is applied to the vessel to sequester the chemibead-sensibead complexes to a wall of the reaction vessel. The vessel is washed to remove unbound material, *i.e.,* excess of unbound sensibead microparticles. The magnetic field is discontinued and the light generation reaction is started, by excitation (680 nm) of the chemibead microparticles. Chemibeads emit light which excites sensibeads (FRET). The fluorescence emitted by sensibead microparticles (520-620 nm) is then measured and quantified by the analyzer.

In a similar implementation, as described above, the second capture system **30'** is directly linked to a sensibead microparticle, instead of indirectly through a specific binding pair. The reaction is carried out as described above, without the addition of the additional reagent (because of the direct sensibead linkage to the second capture system).

### Example 2A - Acridinium labeled nucleic acid for competition assay

Paramagnetic microparticles are used as the support, in order to concentrate the target nucleic acid **40** and competing nucleic acid **40'.** Light is produced by direct excitation of acridinium. Acridinium labeled nucleic acid fragments are added in known concentration to the reaction vessel, with the sample **60** containing target nucleic acid **40.** The reaction mixture incubates with the capture system in the vessel, and the nucleic acid, labeled or not, binds with a capture system, linked, via a linker **20,** to a paramagnetic microparticle, forming a conjugate, *e.g.,* an intercalating complex. After a set incubation time, a magnetic field is applied to the vessel, which attracts the paramagnetic microparticles to a wall of the reaction vessel. The vessel is washed to remove unbound material, *i.e.,* excess of labeled nucleic acid fragments **40'** and other crude components of the sample **60.** The magnetic field is discontinued and the light generation reaction is started, after addition and mixture (either manually or automated) of a specific reagent (*e.g.,* hydrogen peroxide, acidic medium, and then sodium hydroxide or Bayer reagent) that chemically excites acridinium. The luminescence is then measured by the detector of the instrument.

### Example 2B - Nucleic acids indirectly labeled with acridinium

Example 2A can be further modified by using competing labeled nucleic acid **40'** labeled with biotin or avidin, instead of a direct label of acridinium. The competing nucleic acid **40'** is added to the reaction in concert with an avidin or biotin molecule labeled with acridinium, either in an automated fashion or manually. Avidin binds biotin (which is linked to the sequestered competing nucleic acid conjugate). After an incubation time, a magnetic field is applied to the vessel and sequesters the paramagnetic microparticles to a wall of the reaction vessel. The vessel is washed to remove unbound material, *i.e.*, excess of labeled nucleic acid fragments **40'** and unbound luminescent molecules, and other crude components of the sample **60.** The magnetic field is discontinued and the light generation reaction is started, after manual or automated addition of a specific reagent that chemically excites acridinium. The luminescence is then measured by the instrument. The intensity of luminescence is inversely proportional to the nucleic acid concentration in the crude sample **60.**

### Example 2C - XL665 label

The competing nucleic acid **40'** exemplified in Example 2A can be modified to include a XL665 label instead of acridinium. The reaction is carried out in the same fashion as Example 2A, except no additional reagent is added to generate the light emission reaction. The first linker **20** is derivatized with a cryptate molecule. Reaction of light generation is started by the analyzer, by excitation of the cryptate at 337 nm. Cryptate and XL665 are a FRET pair, so excitation of cryptate causes the excitation of XL665. The XL665 fluorescence intensity is measured by the analyzer.

### Example 2D

The reagents in Example 2A can be further modified to include dioxetane phosphate-labeled nucleic acid or ruthenium labeled nucleic acid as the labeled competing nucleic acid **40'.** After wash and supernatant removal steps, the light generation reaction is started by manual or automated addition of a specific Beckman reagent (when using dioxentane - Lumi-Phos 530, alkaline phosphatase, surfactant fluorescein) or a specific Roche reagent (ruthenium-tris(2-pyridylmethyl)amine that serves to oxidize ruthenium). The luminescence is then measured by the analyzer.

### Example 2E - Dade Behring reagents

Labeled nucleic acid fragments **40'** in known concentrations are added to a reaction vessel, along with the sample **60** containing a target nucleic acid **40,** and a first capture system **30.** The reaction mixture incubates and the nucleic acids, labeled or not, bind with a capture system, linked, via a linker **20,** to a chemibead microparticle (*i.e.,* the support **10**). After an incubation time, a magnetic field is applied and sequesters the paramagnetic chemibead microparticles to a wall of the reaction vessel. The vessel is washed to remove unbound material, *i.e.,* excess of labeled nucleic acid fragments **40'.** Label catchers linked to sensibead microparticles are added to the reaction. The label **50** and label catchers can be specific binding pairs, for example, biotin and avidin, respectively. The label catcher reacts with the label **50** linked to the nucleic acid conjugate. After an incubation time, a magnetic field is applied to the vessel and attracts the chemibead-sensibead complexes to a wall of the reaction vessel. The vessel is washed to remove unbound material, *i.e.,* excess of sensibead microparticles. The light generation reaction is started, by excitation (680 nm) of the chemibead microparticles. Chemibeads emit light which in turn electronically excites sensibead microparticles. The fluorescence emitted by sensibead microparticles (520-620 nm) is measured by the analyzer.

### Example 3

The present inventors have found that measurements in the present method are more accurate if a set of two or more interactors is used. Specifically, the inventors have found that the amount of observed fluorescence or other signal used to quantify the amount of the nucleic acid in a sample is dependent on the length and (less so) on the sequence of target nucleic acid present in a sample.

In an experiment employing DNA fragments of 432, about 500, about 800 and 1400 bp, wherein a single interactor was used, fluorescence was measured at 568 nm, 526 nm, 502 nm and both its measured and its calculated total values varied considerably, with longer fragments producing higher values that were four or more times higher than those of the shortest fragment. The variation in fluorescence persisted at different concentrations of fragment. As expected, fluorescence was a linear function of concentration. Fluorescence was also found to vary but less so according to the sequence of the DNA fragment when the size was kept approximately constant. For example in one experiment fragments of 203 bp having disparate sequences produced calculated fluorescence as low as 9.156 and as high as 29.257.

Such differences especially those due to size will be significantly reduced or altogether eliminated by use of dual interactors.

Interactors, that are chemical molecules, may react differently according to length or sequence of nucleic acids. Particularly if interactors are intercalating agents, our experience clearly exhibited such dependence.

We tested intercalating agents with several type of nucleic acid sequences. In this experiment, we measured fluorescence emitted by the DNA/intercalator complex.

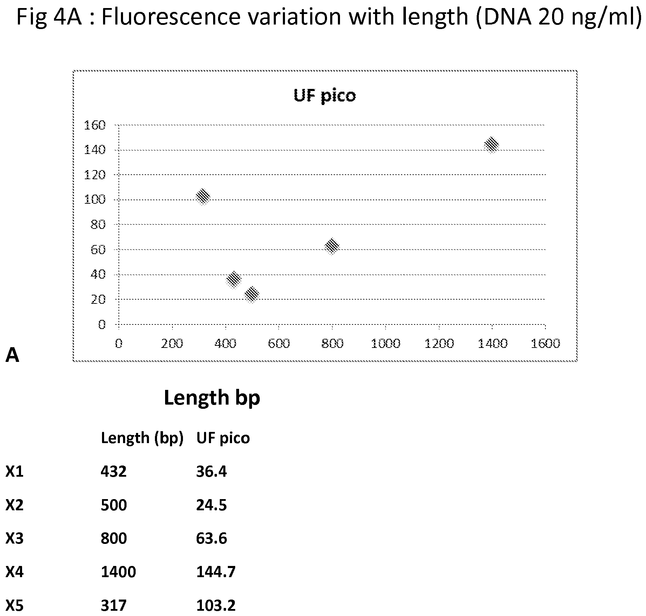

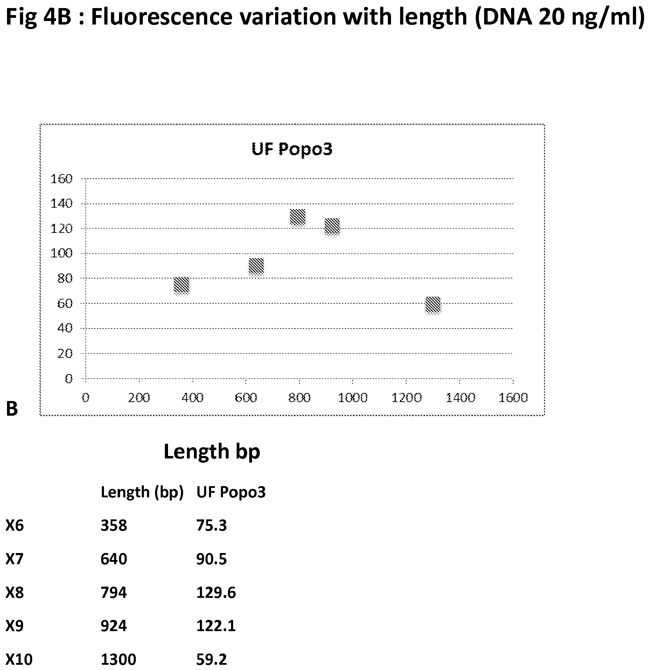

We observed that the fluorescence signal was not constant if we analysed the same concentration of five different sequences of variable lengths (Fig 4A XI-X5). The intercalating agent used was Picogreen. We repeated a similar experiment with another sequence and another intercalating agent, Popo3. Results were drastically different (Fig 4, B X6-X10), indicating that the fluorescence signal varies with the length of the detected DNA.

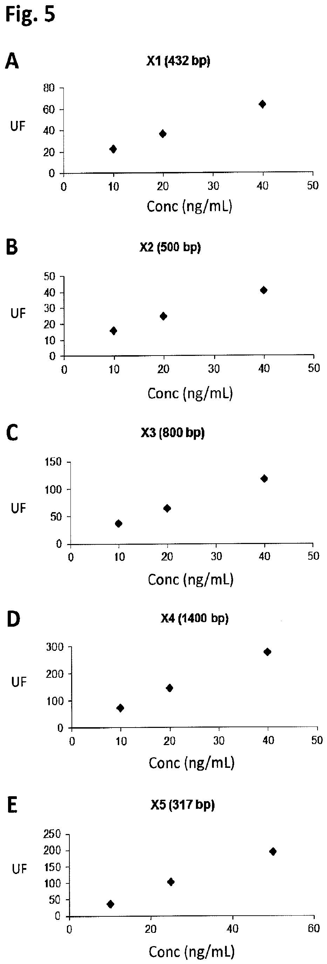

However, when we tested a unique sequence at different concentrations, signal intensity is proportional to nucleic acid concentration. This experience was repeated with several sequences (X1 to X5) used in the previous experiment (Fig 5).

**Table 1:Sequences of X1-X5**

| |
|---|
| X1 - 432 bp |
| actcttctgg tccccacaga ctcagagaga acccaccatg gtgctgtctc ctgccgacaa gaccaacgtc aaggccgcct ggggtaaggt cggcgcgcac gctggcgagt atggtgcgga ggccctggag aggatgttcc tgtccttccc caccaccaag acctacttcc cgcacttcga cctgagccac ggctctgccc aggttaaggg ccacggcaag aaggtggccg acgcgctgac caacgccgtg gcgcacgtgg acgacatgcc caacgcgctg tccgccctga gcgacctgca cgcgcacaag cttcgggtgg acccggtcaa cttcaagctc ctaagccact gcctgctggt gaccctggcc gcccacctcc ccgccgagtt cacccctgcg gtgcacgcct ccctggacaa gttcctggct tc (SEQ ID NO:1) |
| X2 - 500 bp |
| atggcaagaa agtgctcggt gcctttagtg atggcctggc tcacctggac aacctcaagg agtcctttgg ggatctgtcc actcctgatg ctgttatggg caaccctaag gtgaaggctc tcaggctcct gggcaacgtg ctggtctgtg tgctggccca tcactttggc aaagaattca acatttgctt ctgacacaac tgtgttcact agcaacctca aacagacacc atggtgcatc tgactcctga ggagaagtct gccgttactg ccctgtgggg caaggtgaac gtggatgaag ttggtggtga ggccctgggc aggctgctgg tggtctaccc ttggacccag aggttctttg gcacctttgc cacactgagt gagctgcact gtgacaagct gcacgtggat cctgagaact ccccaccagt gcaggctgcc tatcagaaag tggtggctgg tgtggctaat gccctggccc acaagtatca ctaagctcgc(SEQ ID NO:2) |
| X3 - 800 bp |
| tatatgttta ttctttttat catctggaaa cattcattat agttactgtc actaatcctt cacacttcca aaatctgaca agtttgacat aggaaaaaaa tgggggaaat gtagatgaaa gagtttctat catttgaaa attgtgttat aaaaattaag atgtctatcc cctctaggaa ttcctatgag ggatccttca cagtctaaag gaaatgaagt ttgacaatgg tacaattcag ttttgaattt ttgctcttac tttctatatc ctaacctttt ccagcttatt tcttgttaat tgtttttgga aagtatgcaa tgctctttta agggaaaaaa aatcttctaa tgcacttatt taccttcatt tatatgtgtt tgtttctgaa atgaagaatc aagctttggt cattccggaa gtcgtgtagc atgctccaat ttgaagtgac tgagatcttt tgtgacttac agaggagaag gaaatgtttt aaaaattggc ttatgccagt ctccttactc tgaaattctc aattttcttg tattacagga taaatataaa tatcatctca ccaaattgta ccagcattgt cctaaacatt taaattttcc tttatttttg gcctttaaaa ttagaaaatt ttctccagtt gctgcactta gctttttaat tttgttttct tttcacttac cagactactg gtatgatctc tttcactgca gtatgattgc tgctatggtc acttttagag gagattgtgg gaaggttagg atttggagga aacatgacaa aaaagggaag (SEQ ID NO:3) |
| X4 - 1400 bp |
| aagagagagc gcacacgcac acacccgccg cgcgcactcg cgcacggacc cgcacgggga cagctcggaa gtcatcagtt ccatgggcga gatgctgctg ctggcgagat gtctgctgct agtcctcgtc tcctcgctgc tggtatgctc gggactggcg tgcggaccgg gcagggggtt cgggaagagg aggcacccca aaaagctgac ccctttagcc tacaagcagt ttatccccaa tgtggccgag aagaccctag gcgccagcgg aaggtatgaa gggaagatct ccagaaactc cgagcgattt aaggaactca cccccaatta caaccccgac atcatattta aggatgaaga aaacaccgga gcggacaggc tgatgactca gaggtgtaag gacaagttga acgctttggc catctcggtg atgaaccagt ggccaggagt gaaactgcgg gtgaccgagg gctgggacga agatggccac cactcagagg agtctctgca ctacgagggc cgcgcagtgg acatcaccac |
| gtctgaccgc gaccgcagca agtacggcat gctggcccgc ctggcggtgg aggccggctt cgactgggtg tactacgagt ccaaggcaca tatccactgc tcggtgaaag cagagaactc ggtggcggcc aaatcgggag gctgcttccc gggctcggcc acggtgcacc tggagcaggg cggcaccaag ctggtgaagg acctgagccc cggggaccgc gtgctggcgg cggacgacca gggccggctg ctctacagcg acttcctcac tttcctggac cgcgacgacg gcgccaagaa ggtcttctac gtgatcgaga cgcgggagcc gcgcgagcgc ctgctgctca ccgccgcgca cctgctcttt gtggcgccgc acaacgactc ggccaccggg gagcccgagg cgtcctcggg ctcggggccg ccttccgggg gcgcactggg gcctcgggcg ctgttcgcca gccgcgtgcg cccgggccag cgcgtgtacg tggtggccga gcgtgacggg gaccgccggc tcctgcccgc cgctgtgcac agcgtgaccc taagcgagga ggccgcgggc gcctacgcgc cgctcacggc ccagggcacc attctcatca accgggtgct ggcctcgtgc tacgcggtca tcgaggagca cagctgggcg caccgggcct tcgcgccctt ccgcctggcg cacgcgctcc tggctgcact ggcgcccgcg cgcacggacc gcggcgggga cagcggcggc ggggaccgcg ggggcggcgg cggcagagta gccctaaccg ctccaggtgc tgccgacgct ccgggtgcgg gggccaccgc gggcatccac tggtactcgc(SEQ ID NO:4) |
| X5 - 317 bp |
| ggtgagcggc ctccgaagcg gagcggggct ctgaggagac actttttttt tcctccctcc ttccctcctc tcctcctccc ttcccttccc ctctcctccc ctctctcctc cttcccccct cggtccgccg gagcctgctg gggcgagcgg ttggtattgc aggcgcttgc tctccggggc cgcccggcgg gtagctggcg gggggaggag gcaggaaccg cgatggcgcc tcagaagcac ggcggtgggg gagggggcgg ctcggggccc agcgcggggt ccgggggagg cggcttcggg ggttcggcgg cggtggc (SEQ ID NO:5) |

Thus, in light of these experiments, we can conclude that the observed discrepancies are not due to a problem of quantification, but rather to a problem of interaction between intercalators and nucleic acid length and/or sequence. These problems are corrected by use of more than one intercalator, such as two, three four etc.

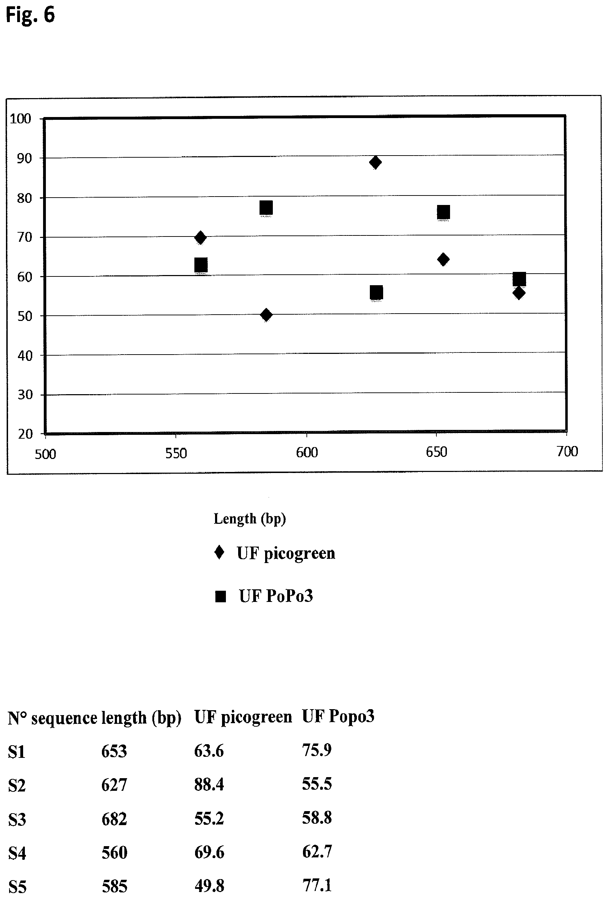

For that purpose, we tested five different sequences of similar lengths, with either Picogreen or Popo3 (Fig 6). We observed a sequence dependence of signal intensity, regardless of which the intercalating agent was used.

**Table 2: Sequences of S1-S5**

| |
|---|
| atggcaagaa agtgctcggt gcctttagtg atggcctggc tcacctggac aacctcaagg agtcctttgg ggatctgtcc actcctgatg ctgttatggg caaccctaag gtgaaggctc tcaggctcct gggcaacgtg ctggtctgtg tgctggccca tcactttggc aaagaattca acatttgctt ctgacacaac tgtgttcact agcaacctca aacagacacc atggtgcatc tgactcctga ggagaagtct gccgttactg ccctgtgggg caaggtgaac gtggatgaag ttggtggtga ggccctgggcaggctgctgg tggtctaccc ttggacccag aggttctttg gcacctttgc cacactgagt gagctgcact gtgacaagct gcacgtggat cctgagaact ccccaccagt gcaggctgcc tatcagaaag tggtggctgg tgtggctaat gccctggccc acaagtatca ctaagctcgc tttcttgctg tccaatttct attaaaggtt cctttgttcc ctaagtccaa ctactaaact gggggatatt atgaagggcc ttgagcatct ggattctgcc taataaaaaa catttatttt agtgctcggt gcctttagtg atggcctggc cgg(SEQ ID NO:6) S1 - 653 bp |
| S2 - 627 bp |
| gcacctttgc cacactgagt gagctgcact gtgacaagct gcacgtggat cctgagaact tcaggctcct gggcaacgtg ctggtctgtg tgctggccca tcactttggc aaagaattca ccccaccagt gcaggctgcc tatcagaaag tggtggctgg tgtggctaat gccctggccc acaagtatca ctaagctcgc tttcttgctg tccaatttct attaaaggtt cctttgttcc acatttgctt ctgacacaac tgtgttcact agcaacctca aacagacacc atggtgcatc tgactcctga ggagaagtct gccgttactg ccctgtgggg caaggtgaac gtggatgaag ttggtggtga ggccctgggc aggctgctgg tggtctaccc ttggacccag aggttctttg agtcctttgg ggatctgtcc actcctgatg ctgttatggg caaccctaag gtgaaggctc atggcaagaa agtgctcggt gcctttagtg atggcctggc tcacctggac aacctcaagg ctaagtccaa ctactaaact gggggatatt atgaagggcc ttgagcatct ggattctgcc taataaaaaa catttatttt cattgct(SEQ ID NO:7) |
| S3 - 682 bp |
| acatttgctt ctgacacaac tgtgttcact agcaacctca aacagacacc atggtgcatc tgactcctga ggagaagtct gccgttactg ccctgtgggg caaggtgaac gtggatgaag ttggtggtga ggccctgggc aggctgctgg tggtctaccc ttggacccag aggttctttg agtcctttgg ggatctgtcc actcctgatg ctgttatggg caaccctaag gtgaaggctc atggcaagaa agtgctcggt gcctttagtg atggcctggc tcacctggac aacctcaagg gcacctttgc cacactgagt gagctgcact gtgacaagct gcacgtggat cctgagaact tcaggctcct gggcaacgtg ctggtctgtg tgctggccca tcactttggc aaagaattca ccccaccagt gcaggctgcc tatcagaaag tggtggctgg tgtggctaat gccctggccc acaagtatca ctaagctcgc tttcttgctg tccaatttct attaaaggtt cctttgttcc ctaagtccaa ctactaaact |
| gggggatatt atgaagggcc ttgagcatct ggattctgcc taataaaaaa catttatttt acatttgctt ctgacacaac tgtgttcact agcaacctca aacagacacc atggtgcatc gc(SEQ ID NO:8) |
| S4 - 560 bp |
| agtcctttgg ggatctgtcc actcctgatg ctgttatggg caaccctaag gtgaaggctc atggcaagaa agtgctcggt gcctttagtg atggcctggc tcacctggac aacctcaagg gcacctttgc cacactgagt gagctgcact gtgacaagct gcacgtggat cctgagaact ttggtggtga ggccctgggc aggctgctgg tggtctaccc ttggacccag aggttctttg tgactcctga ggagaagtct gccgttactg ccctgtgggg caaggtgaac gtggatgaag acatttgctt ctgacacaac tgtgttcact agcaacctca aacagacacc atggtgcatc tcaggctcct gggcaacgtg ctggtctgtg tgctggccca tcactttggc aaagaattca ccccaccagt gcaggctgcc tatcagaaag tggtggctgg tgtggctaat gccctggccc acaagtatca ctaagctcgc tttcttgctg tccaatttct attaaaggtt cctttgttcc ctaagtccaa ctactaaact(SEQ ID NO:9) |
| S5 - 585 bp |
| ttggtggtga ggccctgggc aggctgctgg tggtctaccc ttggacccag aggttctttg tgactcctga ggagaagtct gccgttactg ccctgtgggg caaggtgaac gtggatgaag acatttgctt ctgacacaac tgtgttcact agcaacctca aacagacacc atggtgcatc agtcctttgg ggatctgtcc actcctgatg ctgttatggg caaccctaag gtgaaggctc atggcaagaa agtgctcggt gcctttagtg atggcctggc tcacctggac aacctcaagg gcacctttgc cacactgagt gagctgcact gtgacaagct gcacgtggat cctgagaact tcaggctcct gggcaacgtg ctggtctgtg tgctggccca tcactttggc aaagaattca ccccaccagt gcaggctgcc tatcagaaag tggtggctgg tgtggctaat gccctggccc acaagtatca ctaagctcgc tttcttgctg tccaatttct attaaaggtt cctttgttcc ctaagtccaa ctactaaact gggggatatt atgaagggcc ttgag(SEQ ID NO:10) |

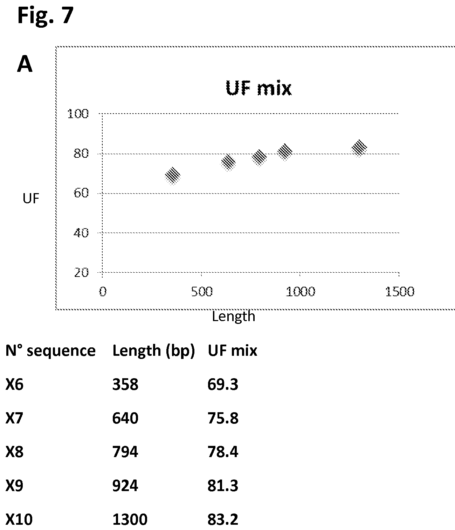

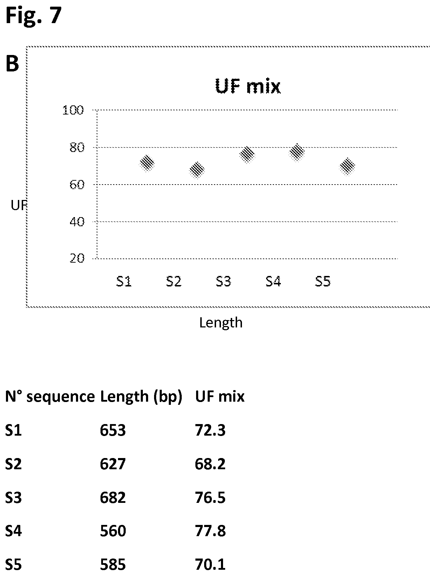

The reduction or elimination of length and sequence dependence of the signal, is illustrated by the use of several intercalating agents : picogreen, popo 3, yoyo 1, orange acridin, at equimolar ratios. Results are shown in Fig 7A (X6-X10) and B(S1-S5). The use of a set of interactors, such as intercalating agents, confers accuracy to the method.

**Table 3:Sequences of X6-X10**

| |
|---|
| X6 - 358 bp |
| atgaccctct ccggcggcgg cagcgccagc gacatgtccg gccagacggt gctgacggcc gaggacgtgg acatcgatgt ggtgggcgag ggcgacgacg ggctggaaga gaaggacagc gacgcaggtt gcgatagccc cgcggggccg ccggagctgc gcctggacga ggcggacgag gtgcccccgg cggcacccca tcacggacag cctcagccgc cccaccagca gcccctgaca ttgcccaagg aggcggccgg agccggggcc ggaccggggg gcgacgtggg cgcgccggag gcggacggct gcaagggcgg tgttggcggc gaggagggcg gcgcgagcgg cggcgggc (SEQ ID NO :11) |
| >X7 - 640 bp |
| gtgcggggga agatgtagca gcttcttctc cgaaccaacc ctttgccttc ggacttctcc ggggccagca gccgcccgac caggggcccg gggccacggg ctcagccgac gaccatgggc tccgtgtcca accagcagtt tgcaggtggc tgcgccaagg cggcagaaga ggcgcccgag gaggcgccgg aggacgcggc ccgggcggcg gacgagcctc agctgctgca cggtgcgggc atctgtaagt ggttcaacgt gcgcatgggg ttcggcttcc tgtccatgac cgcccgcgcc ggggtcgcgc tcgacccccc agtggatgtc tttgtgcacc agagtaagct gcacatggaa gggttccgga gcttgaagga gggtgaggca gtggagttca cctttaagaa gtcagccaag ggtctggaat ccatccgtgt caccggacct ggtggagtat tctgtattgg gagtgagagg cggccaaaag gaaagagcat gcagaagcgc agatcaaaag gagacaggtg ctacaactgt ggaggtctag atcatcatgc caaggaatgc aagctgccac cccagcccaa gaagtgccac ttctgccaga gcatcagcca tatggtagcc tcatgtccgc (SEQ ID NO :12) |
| >X8 - 794 bp |
| attataaatc tagagactcc aggattttaa cgttctgctg gactgagctg gttgcctcat gttattatgc aggcaactca ctttatccca atttcttgat acttttcctt ctggaggtcc tatttctcta acatcttcca gaaaagtctt aaagctgcct taaccttttt tccagtccac ctcttaaatt ttttcctcct cttcctctat actaacatga gtgtggatcc agcttgtccc caaagcttgc cttgctttga agcatccgac tgtaaagaat cttcacctat gcctgtgatt tgtgggcctg aagaaaacta tccatccttg caaatgtctt ctgctgagat gcctcacacg gagactgtct ctcctcttcc ttcctccatg gatctgctta ttcaggacag ccctgattct tccaccagtc ccaaaggcaa acaacccact tctgcagaga agagtgtcgc aaaaaaggaa gacaaggtcc cggtcaagaa acagaagacc agaactgtgt tctcttccac ccagctgtgt gtactcaatg atagatttca gagacagaaa tacctcagcc tccagcagat gcaagaactc tccaacatcc tgaacctcag ctacaaacag gtgaagacct ggttccagaa ccagagaatg aaatctaaga ggtggcagaa aaacaactgg ccgaagaata gcaatggtgt gacgcagaag gcctcagcac ctacctaccc cagcctttac tcttcctacc accagggatg cctggtgaac ccgactggga accttccaat gtggagcaac cagacctgga acaattcaac ctggag(SEQ ID NO :13) |
| >X9 - 924 bp |
| ctggggagag tatatactga atttagcttc tgagacatga tgctcttcct ttttaattaa cccagaactt agcagcttat ctatttctct aatctcaaaa catccttaaa ctgggggtga tacttgagtg agagaatttt gcaggtatta aatgaactat cttctttttt tttttctttt gagacagagt cttgctctgt cacccaggct ggagtgcagt ggcgtgatct cagctcactg caacctccgc ctcccgggtt caagtgattc tcctgcctca gcctcctgag tagctgggat tacaggtgcg tgccaccgtg cccagctaat ttttgtgttt ttagtagaga cggggtttca ccatgttggc catgctggtc ttgaactcct gacctcgtga tctgcccacc tcggcctccc aaagtgctgg aattataggc gtgagccacc gcgcccagca aagaacttct aaccttcata acctgacagg tgttctcgag gccagggtct ctctttctgt cctttcacga tgctctgcat cccttggatg tgccagtttc tgggggaaga gtagtccttt gttacatgca tgagtcagtg aacagggaat gggtgaatga catttgtggg taggttattt ctagaagtta ggtgggcagc ttggaaggca gaggcacttc tacagactat tccttggggc cacacgtagg ttcttgaatc ccgaatggaa aggggagatt gataactggt gtgtttatgt tcttacaagt cttctgcctt ttaaaatcca gtcccaggac atcaaagctc tgcagaaaga actcgagcaa tttgccaagc tcctgaagca gaagaggatc accctgggat atacacaggc cgatgtgggg ctcaccctgg gggttctatt tgggaaggta ttca (SEQ ID NO :14) |
| >X10 - 1300 bp |
| atcagcccac caggagacct cgcccgccgc tcccccgggc tccccggcca tgtctcccgc ccggctccgg ccccgactgc acttctgcct ggtcctgttg ctgctgctgg tggtgccggc ggcatggggc tgcgggccgg gtcgggtggt gggcagccgc cggcgaccgc cacgcaaact cgtgccgctc gcctacaagc agttcagccc caatgtgccc gagaagaccc tgggcgccag cggacgctat gaaggcaaga tcgctcgcag ctccgagcgc ttcaaggagc tcacccccaa ttacaatcca gacatcatct tcaaggacga ggagaacaca ggcgccgacc gcctcatgac ccagcgctgc aaggaccgcc tgaactcgct ggctatctcg gtgatgaacc agtggcccgg tgtgaagctg cgggtgaccg agggctggga cgaggacggc caccactcag aggagtccct gcattatgag ggccgcgcgg tggacatcac cacatcagac cgcgaccgca ataagtatgg actgctggcg cgcttggcag tggaggccgg ctttgactgg gtgtattacg agtcaaaggc ccacgtgcat tgctccgtca agtccgagca ctcggccgca gccaagacgg gcggctgctt ccctgccgga gcccaggtac gcctggagag tggggcgcgt gtggccttgt cagccgtgag gccgggagac cgtgtgctgg ccatggggga ggatgggagc cccaccttca gcgatgtgct cattttcctg gaccgcgagc ctcacaggct gagagccttc caggtcatcg agactcagga ccccccacgc cgcctggcac tcacacccgc tcacctgctc tttacggctg acaatcacac ggagccggca gcccgcttcc gggccacatt tgccagccac gtgcagcctg gccagtacgt gctggtggct ggggtgccag gcctgcagcc tgcccgcgtg gcagctgtct ctacacacgt ggccctcggg gcctacgccc cgctcacaaa gcatgggaca ctggtggtgg aggatgtggt ggcatcctgc ttcgcggccg tggctgacca ccacctggct cagttggcct tctggcccct gagactcttt cacagcttgg catggggcag ctggaccccg ggggagggtg tgcattggta cccccagctg ctctaccgcc tggggcgtct cctgctagaa gagggcagct tccacccact gggcatgtcc ggggcaggga gctgaaagga ctccaccgct (SEQ ID NO :15) |

Although the invention has been described in terms of exemplary embodiments, it is not limited thereto. The claims should be construed broadly to include other embodiments of the invention that can be made by ones of ordinary skill in the art in light of the present disclosure.

The invention is further described below by reference to claims as follows:

## Claims

1. A method for quantifying a cell free target nucleic acid in a sample comprising the steps of:
(a) contacting the cell free target nucleic acid in the sample with at least one non sequence specific nucleic acid interactor to form a conjugate;
(b) sequestering the conjugate by reacting the conjugate with a capture molecule bound to a support, wherein the capture molecule bound to the support is a nucleic acid intercalator;
(c) labeling the sequestered conjugate with a signal generating molecule to form a labeled conjugate;
(d) measuring signal of the labeled conjugate; wherein the signal of the labeled conjugate is indicative of the amount of the target nucleic acid in the sample.

2. A method for quantifying a cell free target nucleic acid in a sample comprising the steps of:
(a) providing a known quantity of nucleic acid labeled with a signal generating molecule into the sample containing the target nucleic acid;
(b) reacting the cell free target nucleic acid and the labeled nucleic acid with a sequestered non sequence specific nucleic acid interactor , to form a sequestered target conjugate and a sequestered labeled conjugate, wherein said non sequence specific interactor is an intercalator, wherein the target nucleic acid competes with the labeled nucleic acid for binding to said sequestered intercalactor;
(c) measuring the signal of the sequestered labeled conjugate; and
(d) determining the amount of said target nucleic acid by comparing the signal in step (c) with that detected by sequestered labeled conjugate in the absence of target nucleic acid.

3. A method for quantifying a cell free target nucleic acid in a sample comprising the steps of:
(a) contacting the target nucleic acid in the sample with a sequestered non sequence specific nucleic acid interactor to form a sequestered conjugate, wherein said non sequence specific nucleic acid interactor is an intercalator,;
(b) contacting the sequestered conjugate with a second non sequence specific nucleic acid interactor labeled with a signal generating element to form a labeled conjugate; and
(c) measuring signal of the labeled conjugate; wherein the signal of the labeled conjugate is indicative of the amount of the target nucleic acid in the sample.

4. The method of anyone of claims 1-3, wherein steps (a) and (b) are carried out in the same vessel.

5. The method of anyone of claims 1-4 wherein said nonsequence specific nucleic acid interactor is a set of at least two nucleic acid interactors .

6. The method of anyone of claims 1-5, wherein the sample comprises molecules other than nucleic acids.

7. The method of claim 1, wherein the support is a magnetic particle.

8. The method of claim 7, wherein the magnetic particle is a ferromagnetic particle, paramagnetic particle, superparamagnetic particle, paramagnetic microparticle, or paramagnetic chemibead microparticle.

9. The method of anyone of claims 1 to 8 wherein the nucleic acid has not been isolated or purified.

10. The method of claim 3, wherein steps (a), (b) and (c) are carried out in the same vessel.

11. The method of anyone of claims 1 to 10 wherein the interactor is selected from the group consisting of acridine, picogreen, pop03, yo yo 1, and combinations thereof.

12. The method of anyone of claims 1 to 11, wherein the target nucleic acid is double-stranded nucleic acid.

13. A kit for quantifying a target nucleic acid in a sample comprising
(i) a first nucleic acid interactor that is a nucleic acid intercalator;
(ii) a second nucleic acid interactor that is labeled with a readable label and which interactor is different from said first nucleic acid interactor;
(iii) a label, optionally bound to the first interactor;
(iv) instructions for use, and
optionally, further comprising control nucleic acid fragments of different sizes and sequences.

14. A kit as claimed in claim 13 wherein said first nucleic acid interactor is bound to a support

## Patentansprüche

1. Verfahren zur Quantifizierung einer zellfreien Zielnukleinsäure in einer Probe umfassend die Schritte:
(a) Kontaktieren der zellfreien Zielnukleinsäure in der Probe mit mindestens einem nicht-sequenzspezifischen Nukleinsäureinteraktor unter Bildung eines Konjugats;
(b) Sequestrieren des Konjugats durch Reagieren des Konjugats mit einem Fängermolekül, welches an einen Träger gebunden ist, wobei das an einen Träger gebundene Fängermolekül ein Nukleinsäureinterkalator ist;
(c) Markieren des sequestrierten Konjugats mit einem ein Signal generierenden Molekül unter Bildung eines markierten Konjugats;
(d) Messen des Signals des markierten Konjugats; wobei das Signal des markierten Konjugats bezeichnend für die Menge der Zielnukleinsäure in der Probe ist.

2. Verfahren zur Quantifizierung einer zellfreien Zielnukleinsäure in einer Probe umfassend die Schritte:
(a) Bereitstellen einer bekannten Menge einer Nukleinsäure, welche mit einem ein Signal erzeugenden Molekül markiert ist, in der Probe, welche die Zielnukleinsäure enthält;
(b) Reagieren der zellfreien Zielnukleinsäure und der markierten Nukleinsäure mit einem sequestrierten nicht-sequenzspezifischen Nukleinsäureinteraktor unter Bildung eines sequestrierten Zielkonjugats und eines sequestrierten markierten Konjugats, wobei der nichtsequenzspezifische Interaktor ein Interkalator ist, worin die Zielnukleinsäure mit der markierten Nukleinsäure um die Bindung des sequestrierten Interkalators kompetiert;
(c) Messen des Signals des sequestrierten markierten Konjugats; und
(d) Bestimmen der Menge der Zielnukleinsäure durch Vergleichen des Signals in Schritt c) mit denjenigen, welches für das sequestrierte markierte Konjugat in Abwesenheit der Zielnukleinsäure bestimmt wurde.

3. Verfahren zur Quantifizierung einer zellfreien Zielnukleinsäure in einer Probe umfassend die Schritte:
(a) Inkontaktbringen der Zielnukleinsäure in der Probe mit einem sequestrierten nicht-sequenzspezifischen Nukleinsäureinteraktor unter Bildung eines sequestrierten Konjugats, worin der nichtsequenzspezifische Nukleinsäureinteraktor ein Interkalator ist;
(b) Kontaktieren des sequestrierten Konjugats mit einem zweiten nicht-sequenzspezifischen Nukleinsäureinteraktor, welcher mit einem signalerzeugenden Element markiert ist, unter Bildung eines markierten Konjugats, und
(c) Messen des Signals des markierten Konjugats; wobei das Signal des markierten Konjugats bezeichnend für die Menge der Zielnukleinsäure in der Probe ist.

4. Verfahren gemäß einem der Ansprüche 1-3, worin die Schritte (a) und (b) in demselben Gefäß ausgeführt werden.

5. Verfahren gemäß einem der Ansprüche 1-4, worin der nichtsequenzspezifische Nukleinsäureinteraktor ein Set von mindestens zwei Nukleinsäureinteraktoren ist.

6. Verfahren gemäß einem der Ansprüche 1-5, worin die Probe andere Moleküle als Nukleinsäuren umfasst.

7. Verfahren gemäß Anspruch 1, worin der Träger ein magnetisches Teilchen ist.

8. Verfahren gemäß Anspruch 7, worin das magnetische Teilchen ein ferromagnetisches Teilchen, paramagnetisches Teilchen, superparamagnetisches Teilchen, paramagnetisches Mikroteilchen oder paramagnetisches Chemibead-Mikroteilchen ist.

9. Verfahren gemäß einem der Ansprüche 1-8, worin die Nukleinsäure nicht isoliert oder gereinigt wurde.

10. Verfahren nach Anspruch 3, worin die Schritte (a), (b) und (c) in demselben Gefäß ausgeführt werden.

11. Verfahren gemäß einem der Ansprüche 1-10, worin der Interaktor ausgewählt ist aus der Gruppe bestehend aus Acridin, PicoGreen, pop03, yo yo 1 und Kombinationen hiervon.

12. Verfahren gemäß einem der Ansprüche 1-11, wobei die Zielnukleinsäure eine doppelsträngige Nukleinsäure ist.

13. Kit zur Quantifizierung einer Zielnukleinsäure in einer Probe umfassend
(i) einen ersten Nukleinsäureinteraktor, der ein Nukleinsäureinterkalator ist;
(ii) einen zweiten Nukleinsäureinteraktor, der mit einer lesbaren Markierung markiert ist und welcher Interaktor verschieden von dem ersten Nukleinsäureinteraktor ist;
(iii) eine Markierung, gegebenenfalls gebunden an den ersten Interaktor;
(iv) Verwendungsinstruktionen, und
gegebenenfalls weiter umfassend Kontrollnukleinsäurefragmente unterschiedlicher Größen und Sequenzen.

14. Kit gemäß Anspruch 13, worin der erste Nukleinsäureinteraktor an einen Träger gebunden ist.

## Revendications

1. Procédé de quantification d'un acide nucléique cible exempt de cellule dans un échantillon comprenant les étapes de :
(a) mise en contact de l'acide nucléique cible exempt de cellule dans l'échantillon avec au moins un interacteur d'acide nucléique non spécifique de séquence pour former un conjugué ;
(b) séquestration du conjugué par réaction du conjugué avec une molécule de capture liée à un support, où la molécule de capture liée au support est un intercalateur d'acide nucléique ;
(c) étiquetage du conjugué séquestré avec une molécule de génération de signal pour former un conjugué marqué ;
(d) mesure du signal du conjugué marqué ; où le signal du conjugué marqué est indicateur de la quantité d'acide nucléique cible dans l'échantillon.

2. Procédé de quantification d'un acide nucléique cible exempt de cellule dans un échantillon comprenant les étapes de :
(a) fourniture d'une quantité connue d'un acide nucléique marqué avec une molécule de génération de signal dans l'échantillon contenant l'acide nucléique cible ;
(b) réaction de l'acide nucléique cible exempt de cellule et de l'acide nucléique marqué avec un interacteur séquestré d'un acide nucléique non spécifique de séquence, pour former un conjugué cible séquestré et un conjugué marqué séquestré, où ledit interacteur non spécifique de séquence est un intercalateur, où l'acide nucléique cible entre en compétition avec l'acide nucléique marqué pour la liaison audit intercalateur séquestré ;
(c) mesure du signal du conjugué marqué séquestré ; et
(d) détermination de la quantité dudit acide nucléique cible en comparant le signal dans l'étape (c) à celui détecté par le conjugué marqué séquestré en l'absence d'acide nucléique cible.

3. Procédé de quantification d'un acide nucléique cible exempt de cellule dans un échantillon comprenant les étapes de :
(a) mise en contact de l'acide nucléique cible dans l'échantillon avec un interacteur séquestré d'acide nucléique non spécifique de séquence pour former un conjugué séquestré, où ledit interacteur d'acide nucléique non spécifique de séquence est un intercalateur ;
(b) mise en contact du conjugué séquestré avec un second interacteur d'acide nucléique non spécifique de séquence marqué avec un élément de génération de signal pour former un conjugué marqué ; et
(c) mesure du signal du conjugué marqué ; où le signal du conjugué marqué est indicateur de la quantité d'acide nucléique cible dans l'échantillon.

4. Procédé selon l'une quelconque des revendications 1 à 3, dans lequel les étapes (a) et (b) sont conduites dans le même récipient.

5. Procédé selon l'une quelconque des revendications 1 à 4 dans lequel ledit interacteur d'acide nucléique non spécifique de séquence est un ensemble d'au moins deux interacteurs d'acide nucléique.

6. Procédé selon l'une quelconque des revendications 1 à 5, dans lequel l'échantillon comprend des molécules autres que des acides nucléiques.

7. Procédé selon la revendication 1, dans lequel le support est une particule magnétique.

8. Procédé selon la revendication 7, dans lequel la particule magnétique est une particule ferromagnétique, une particule paramagnétique, une particule superparamagnétique, une microparticule paramagnétique, ou une microparticule paramagnétique type chemibead.

9. Procédé selon l'une quelconque des revendications 1 à 8 dans lequel l'acide nucléique n'a été ni isolé ni purifié.

10. Procédé selon la revendication 3, dans lequel les étapes (a), (b) et (c) sont conduites dans le même récipient.

11. Procédé selon l'une quelconque des revendications 1 à 10 dans lequel l'interacteur est sélectionné dans le groupe constitué de l'acridine, du picogreen, du pop 03, du YOYO 1, et de leurs combinaisons.

12. Procédé selon l'une quelconque des revendications 1 à 11, dans lequel l'acide nucléique cible est l'acide nucléique double brin.

13. Trousse de quantification d'un acide nucléique cible dans un échantillon comprenant
(i) un premier interacteur d'acide nucléique qui est un intercalateur d'acide nucléique ;
(ii) un second interacteur d'acide nucléique qui est marqué avec un marqueur pouvant être lu et lequel interacteur étant différent dudit premier interacteur d'acide nucléique ;
(iii) une étiquette, éventuellement liée au premier interacteur ;
(iv) des instructions d'utilisation, et
éventuellement, comprenant en outre des fragments d'acide nucléique témoins de différentes tailles et séquences.

14. Trousse telle que revendiquée selon la revendication 13 dans laquelle ledit premier interacteur d'acide nucléique est lié à un support.
